(19) Europäisches Patentamt
European Patent Office
Office européen des brevets

(11) Veröffentlichungsnummer: **0 014 928**
**B1**

(12) # EUROPÄISCHE PATENTSCHRIFT

(45) Veröffentlichungstag der Patentschrift: **05.05.82**

(51) Int. Cl.³: **C 07 D 401/12**, C 07 D 401/14, C 07 D 405/14, A 61 K 31/445 // C07D211/22

(21) Anmeldenummer: **80100670.1**

(22) Anmeldetag: **11.02.80**

(54) **Neue Piperidinopropylderivate, Verfahren zu ihrer Herstellung und diese Verbindungen enthaltende Arzneimittel.**

(30) Priorität: **16.02.79 DE 2905876**

(43) Veröffentlichungstag der Anmeldung: **03.09.80 Patentblatt 80/18**

(45) Bekanntmachung des Hinweises auf die Patenterteilung: **05.05.82 Patentblatt 82/18**

(84) Benannte Vertragsstaaten: **AT BE CH DE FR GB IT LU NL SE**

(56) Entgegenhaltungen:
**DE-A-2 550 001**
**DE-A-2 737 630**

(73) Patentinhaber: **BOEHRINGER MANNHEIM GMBH, Sandhofer Strasse 112-132 Postfach 31 01 20, D-6800 Mannheim 31-Waldhof (DE)**

(72) Erfinder: **Friebe, Walter-Gunar, Dr.rer.nat., Heidelberger Landstrasse 111d, D-6100 Darmstadt (DE)**
Erfinder: **Michel, Helmut, Ziegelgasse 2a, D-6800 Mannheim 31 (DE)**
Erfinder: **Ross, Carl Heinz, Dr.rer.nat., Rathausstrasse 45, D-6806 Viernheim (DE)**
Erfinder: **Wiedemann, Fritz, Dr.phil., Weinheimer Strasse 82, D-6940 Weinheim-Lützelsachsen (DE)**
Erfinder: **Sponer, Gisbert, Dr.med.vet., Tilsiterstrasse 30, D-6944 Hemsbach (DE)**
Erfinder: **Schaumann, Wolfgang, Prof.Dr.med., Mönchhofstrasse 58, D-6900 Heidelberg (DE)**

(74) Vertreter: **Weber, Manfred, Dr., Boehringer Mannheim GmbH Patentabteilung Sandhofer Strasse 116, D-6800 Mannheim 31 (DE)**

**EP 0 014 928 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue Piperidinopropylderivate, Verfahren zu ihrer Herstellung und diese Verbindungen enthaltende Arzneimittel

Die vorliegende Erfindung betrifft Piperidinopropylderivate der allgemeinen Formel I

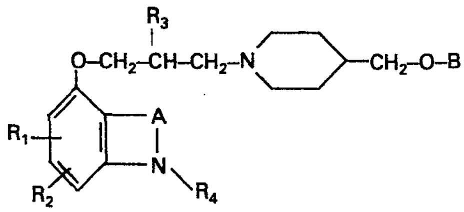

(I),

in der

$R_1$ und $R_2$, die gleich oder verschieden sein können, jeweils ein Wasserstoffatom oder eine $C_1$–$C_6$-Alkylgruppe oder gemeinsam einen $C_2$–$C_4$-Alkylenrest,

$R_3$ Wasserstoff oder eine Gruppe $-O-R_5$, wobei $R_5$ für Wasserstoff oder einen Acetyl-, Pivaloyl- und Benzoylrest steht,

$R_4$ Wasserstoff oder den Formylrest,

A die Gruppe $X_1=Y_1$, wobei $X_1$ und $Y_1$ gleich oder verschieden sein können und jeweils ein Stickstoffatom oder eine Gruppe $-C(R_6)=$ bedeuten, in der $R_6$ für Wasserstoff, eine $C_1$–$C_6$-Alkylgruppe, die gegebenenfalls durch eine Gruppe $-O-R_5$ substituiert ist, eine Carboxyl- oder $C_1$–$C_6$-Alkoxycarbonylgruppe steht, die Gruppe $X_2$–$Y_2$, in der $X_2$ eine $>CH_2$-Gruppe oder eine Gruppe $>N-R_7$, wobei $R_7$ für Wasserstoff oder eine $C_1$–$C_6$-Alkylgruppe steht, und $Y_2$ eine $>CH_2$-Gruppe oder eine Gruppe $>C=Z$, wobei Z für ein Sauerstoff- oder Schwefelatom steht, darstellt, oder die Gruppe $-CR_8=CR_9-$, wobei $R_8$ und $R_9$ zusammen eine $-CH=CH-CH=CH$-Brücke darstellen, mit der Massgabe, dass jeweils $Y_1$ bzw. $Y_2$ mit dem Rest $>N-R_4$ der allgemeinen Formel I verbunden ist, und

B einen Pyridyl-, Pyrimidyl-, Benzimidazolinonyl-, Benzimidazolinyl-, Benztriazolyl-, Indazolyl-, Benzodioxolanyl- oder Carbazolylrest oder, falls A $X_2$–$Y_2$ oder $-CR_8=CR_9-$ darstellt, auch einen Phenylrest, die gegebenenfalls ein- oder mehrfach durch ein Halogen, eine Hydroxy- oder $C_1$–$C_6$-Alkylgruppe, die gegebenenfalls einen Hydroxy- oder Carboxamido-Substituenten trägt, eine $C_1$–$C_6$-Alkoxy-, Amino-, Carboxamido-, $C_1$–$C_6$-Alkylcarbonylamino-, oder $C_1$–$C_6$-Alkylsulfonylaminogruppen substituiert sind, bedeuten, deren pharmakologisch unbedenklichen Salze, Verfahren zur Herstellung derselben sowie pharmazeutische Zubereitungen, die Substanzen der Formel I enthalten.

Da die Verbindungen der Formel I für den Fall, dass $R_3$ nicht Wasserstoff ist, ein asymmetrisches Kohlenstoffatom besitzen, können sie in optisch aktiver Form oder als racemisches Gemisch vorliegen. Gegenstand der vorliegenden Anmeldung sind sowohl die racemischen Formen als auch die optischen Isomeren.

Die $C_1$–$C_6$-Alkyl- bzw. -Alkoxygruppen, die in den Definitionen der Substituenten $R_1$, $R_2$, $R_5$, $R_6$, $R_7$ und B auftreten, können geradkettig oder verzweigt sein und enthalten vorzugsweise 1–4 Kohlenstoffatome. Bevorzugt sind der Methyl-, Propyl- und tert.-Butylrest bzw. die Methoxy-, Ethoxy-, Propoxy- und n-Butoxygruppe.

Unter Halogen wird im Sinne der Erfindung Fluor, Chlor, Brom und Jod verstanden, insbesondere Fluor, Chlor und Brom.

Unter den heterocyclischen Resten B sind der Pyridyl-, Pyrimidyl-, Benzimidazolinonyl-und Benzodioxylanylrest besonders bevorzugt.

Heterocyclische Reste, die der Benzolring mit der Gruppierung $-A-N(R_4)-$ bildet, sind vorwiegend der Benzimidazolinon-(2)-, Benzimidazolinthion-(2)-, Oxindol-, Indazol-, Carbazol-, Benztriazol-, Benzimidazol-, Indolin- und Indol-Rest.

Die Verbindungen der allgemeinen Formel I sowie ihre pharmakologisch unbedenklichen Salze hemmen adrenergische β-Rezeptoren und senken gleichzeitig in hohem Masse den Blutdruck. Sie eignen sich daher zur Behandlung oder Prophylaxe bei Herz- und Kreislauferkrankungen.

In der DE–OS 27 37 630 sind Verbindungen ähnlicher Struktur und Wirkung beschrieben und beansprucht. Durch Abänderung der heterocyclischen Phenolteile sowie durch Einführung von Heterocyclen in die Seitenkette wurde eine überraschende Wirkungsverbesserung erzielt.

Die Herstellung der neuen Verbindungen der allgemeinen Formel I ist dadurch gekennzeichnet, dass man in an sich bekannter Weise entweder

a) eine Verbindung der allgemeinen Formel II

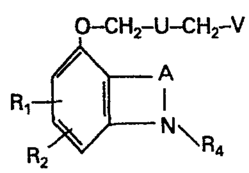

(II)

mit einer Verbindung der allgemeinen Formel III

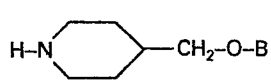

(III)

umsetzt,

in denen $R_1$, $R_2$, $R_4$, A und B die oben angegebene Bedeutung haben, V einen reaktiven Rest darstellt und U für die Gruppe $>C=O$, $>CH-R_3$ oder $>CH-O-E$ steht, wobei $R_3$ die oben angegebene Bedeutung hat und E zusammen mit V eine Einfachbindung bildet, und falls U die Gruppe $>C=O$ bedeutet, anschliessend reduziert, oder

b) eine Verbindung der allgemeinen Formel IV

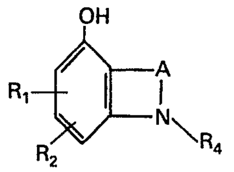

(IV)

mit einer Verbindung der allgemeinen Formel V

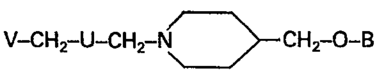

(V)

umsetzt,
in denen $R_1$, $R_2$, $R_4$, A, B, U und V die oben angegebene Bedeutung haben, und falls U die Gruppe >C=O bedeutet, anschliessend reduziert, oder

c) eine Verbindung der allgemeinen Formel VI

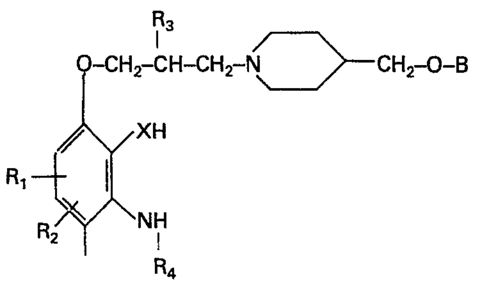

(VI),

in der $R_1$, $R_2$, $R_3$, $R_4$ und B die oben angegebene Bedeutung haben und X $HX_1$ oder $X_2$ vorstellt, für den Fall, dass A eine $-X_1 = Y_1$-Gruppe bedeutet, eine Verbindung der Formel VI, in der X die Gruppe $HX_1$ darstellt, mit einer Verbindung der allgemeinen Formel VII a

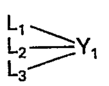

(VII a),

in der $Y_1$ die oben angegebene Bedeutung hat, $L_1$ ein Wasserstoffatom, die Hydroxygruppe oder einen reaktiven Rest T, $L_2$ ein Wasserstoffatom oder einen reaktiven Rest T und $L_3$ ein Wasserstoffatom oder zusammen mit $L_2$ ein Sauerstoffatom darstellt, oder für den Fall, dass A eine $-X_2 - Y_2$-Gruppe bedeutet, eine Verbindung der Formel VI, in der X die Gruppe $X_2$ darstellt, mit einer Verbindung der allgemeinen Formel VII b

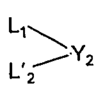

(VII B),

in der $L_1$ und $Y_2$ die oben angegebene Bedeutung haben und $L'_2$ ein Wasserstoffatom oder einen reaktiven Rest T bedeutet, umsetzt und cyclisiert, oder

d) eine Verbindung der allgemeinen Formel VIII

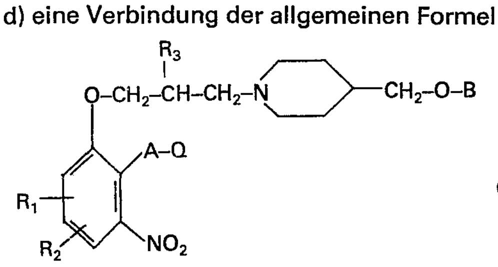

(VIII),

in der $R_1$, $R_2$, $R_3$, A und B die oben angegebene Bedeutung haben und Q eine abspaltbare Gruppe darstellt, reduziert und cyclisiert, und anschliessend Verbindungen der allgemeinen Formel I, in denen $R_3$ die Hydroxygruppe bedeutet, gegebenenfalls acyliert, einen Rest $R_6$ oder einen Substituenten im Rest B gewünschtenfalls in einen anderen Substituenten umwandelt und gegebenenfalls die erhaltenen Verbindungen der allgemeinen Formel I in ihre pharmakologisch verträglichen Salze überführt.

Q, V und T in Verbindungen der allgemeinen Formeln II, V, VII a, VII b und VIII stehen für alle Reste, die sich nucleophil substituieren lassen. Solche Reste sind z.B. Halogenatome, vorzugsweise Brom oder Chlor, Sulfonsäureester-, Amino-, Imidazolyl-, Nieder-alkoxy- oder auch Mercaptogruppen.

Die erfindungsgemässen Verfahren werden zweckmässig in einem unter den Reaktionsbedingungen inerten Lösungsmittel, z.B. Wasser, Ethanol, Dioxan oder Dimethylformamid, gegebenenfalls in Gegenwart eines säurebindenden Mittels durchgeführt. Die Umsetzungen können auch nach Mischen der Reaktionskomponenten ohne Lösungsmittel erreicht werden. Die Reaktionen werden durch Stehenlassen bei Raumtemperatur oder durch Erwärmen, gegebenenfalls unter einer Schutzgasatmosphäre ausgeführt.

Die Umsetzung der Verbindungen der Formel IV mit den Substanzen der Formel V gemäss Verfahren b) erfolgt zweckmässig unter Sauerstoffausschluss in Gegenwart eines Säureakzeptors. Man kann aber auch Alkalisalze der Hydroxyverbindungen der Formel IV einsetzen.

Die gegebenenfalls durchzuführende Reduktion der Gruppe >C=O erfolgt durch katalytische Hydrierung mit Edelmetall- oder Nickelkatalysatoren oder mittels komplexer Metallhydride, wie z.B. Natriumborhydrid.

Verbindungen der allgemeinen Formel III sind in der DE-OS 25 49 999 beschrieben oder können nach dort angegebenen Verfahren hergestellt werden.

Verbindungen der allgemeinen Formel IV sind bekannt oder können analog Verfahren c) oder d) aus bekannten Verbindungen hergestellt werden.

Verbindungen der allgemeinen Formel VI sind aus der Anmeldung P 27 37 630.3 bekannt oder können nach dort angegebenen Verfahren hergestellt werden.

Als Verbindungen der allgemeinen Formel VII a kommen beispielsweise Carbonsäure, wie Ameisensäure oder Essigsäure, Carbonsäureester, Carbonsäurehalogenide in Frage. Als Verbindungen der allgemeinen Formel VII b sind beispielhaft Phosgen, Harnstoff, N,N'-Carbonylbisimidazol, Thiocarbonylhalogenide, Thioharnstoff oder auch Xanthogenate zu nennen. Verbindungen der allgemeinen Formel VII a können auch in situ aus anderen Verbindungen, z.B. anorganischem Nitrit in wässriger Mineralsäure oder Niederalkylsalpetrigsäureester in organischen Lösungsmitteln in der Reaktionsmischung hergestellt werden. Verbindungen VII b können ebenfalls in der Reaktionsmischung, beispielsweise aus Schwefelkohlenstoff in alkalischer Lösung, erzeugt werden.

Reduktionen, wie sie für Verfahren d) notwendig sind, werden vorzugsweise mit katalytisch erregtem Wasserstoff durchgeführt.

Die gegebenenfalls durchzuführende nachträgliche Acylierung von Verbindungen der allgemeinen Formel I, in der $R_3$ eine Hydroxygruppe bedeutet, kann in üblicher Weise durch Umsetzung mit einem reaktiven Säurederivat, z.B. Säurehalogenid, Säureazid oder Säureanhydrid, gegebenenfalls in Gegenwart eines säurebindenden Mittels, z.B. Pyridin, in einem Lösungsmittel, z.B. Aceton, Benzol, Dimethylformamid oder auch in überschüssiger Säure, erfolgen.

Als nachträgliche Umwandlung eines Substituenten $R_6$ in einen anderen Substituenten $R_6$ kommt beispielsweise die Reduktion einer Alkoxycarbonylgruppe zu einem Hydroxymethylrest in Betracht. Diese Reduktion kann nach allgemeinen Methoden durchgeführt werden. Vorzugsweise wird der Carbonsäureester mit komplexen Metallhydriden, beispielsweise Lithiumaluminiumhydrid, in einem neutralen organischen Lösungsmittel, z.B. Tetrahydrofuran, reduziert.

Als nachträgliche Umwandlung eines Substituenten in dem Rest B sei z.B. die Überführung einer Aminogruppe in eine Alkylcarbonylamido- bzw. in eine Alkylsulfonylamidogruppe erwähnt. Diese Umsetzungen erfolgen ebenfalls nach bekannten Methoden mit üblichen Acylierungsmitteln, wie z.B. Carbonsäureanhydriden, Carbonsäurechloriden bzw. Alkylsulfonsäurechloriden.

Die erfindungsgemässen Verbindungen der Formel I können in Form eines racemischen Gemisches anfallen. Die Trennung des Racemats in die optisch aktiven Formen geschieht nach an sich bekannten Methoden über die diastereomeren Salze aktiver Säuren, wie z.B. Weinsäure, Apfelsäure oder Camphersulfonsäure.

Die neuen Verbindungen der allgemeinen Formel I fallen unter den Reaktionsbedingungen der beschriebenen Verfahren vorwiegend als Säureadditionssalze an, z.B. als Hydrochloride, und können nach beschriebenen Methoden ohne weiteres in die freien Basen überführt werden.

Zur Überführung der Verbindungen der allgemeinen Formel I in ihre pharmakologisch unbedenklichen Salze setzt man diese, vorzugsweise in einem organischen Lösungsmittel, mit der äquivalenten Menge einer anorganischen oder organischen Säure, z.B. Salzsäure, Bromwasserstoffsäure, Phosphorsäure, Schwefelsäure, Essigsäure, Citronensäure, Maleinsäure um.

Zur Herstellung von Arzeimitteln werden die Substanzen I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die erfindungsgemässen neuen Substanzen I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Ethanol, Komplexbildner (wie Ethylendiamintetraessigsäure und deren nichttoxische Salze), hochmolekulare Polymere (wie flüssiges Polyethylenoxyd) zur Viskositätsregulierung. Feste Trägerstoffe sind z.B. Stärke, Lactose, Mannit, Methylcellulose, Talkum, hochdisperse Kieselsäuren, hochmolekulare Fettsäuren (wie Stearinsäure), Gelatine, Agar-Agar, Calciumphosphat, Magnesiumstearat, tierische und pflanzliche Fette und feste hochmolekulare Polymere (wie Polyethylenglykole). Für orale Applikation geeignete Zubereitungen können gewünschtenfalls Geschmacks- und Süssstoffe enthalten. Die verabreichte Dosierung hängt vom Alter, der Gesundheit und dem Gewicht des Empfängers, dem Ausmass der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0,1 bis 50 mg/kg Körpergewicht. Normalerwweise sind 0,5 bis 40 und vorzugsweise 1,0 bis 20 mg/kg/Tag in einer oder mehreren Anwendungen pro Tag wirksam, um die gewünschten Resultate zu erhalten.

Bevorzugt im Sinne der vorliegenden Anmeldung ist ausser den in den Beispielen genannten Verbindungen die folgende:

4-[2-Hydroxy-3-(4-phenoxymethylpiperidino)-propoxy]-6,7-cyclopenteno-2-benzimidazolinen

Die Erfindung wird durch die folgenden Beispiele näher erläutert. Sie zeigen einige der zahlreichen möglichen Verfahrensvarianten, die zur Synthese der erfindungsgemässen Verbindungen verwendet werden können. Sie stellen jedoch keine Einschränkung des Erfindungsgegenstandes dar.

Beispiel 1

4-[2-Hydroxy-3-(4-phenoxymethylpiperidino)-propoxy]-2-benzimidazolinon-hydrochlorid

23,0 2,3-Diamino-1-[2-hydroxy-3-(4-phenoxymethylpiperidino)-propoxy]-benzol-trihydrochlorid werden in 600 ml Wasser gelöst. In die Lösung leitet man 40 Minuten Phosgen ein. Das Kristallisat wird abgesaugt, in 500 ml heissem Ethanol aufgenommen, mit Aktivkohle behandelt und mit 2,5 Liter Ether gefällt. Nach nochmaliger Umkristallisation aus Ethanol/Wasser 1:1 erhält man 9.1 g (43% d.Th.) Hydrochlorid der Titelverbindung vom Schmelzpunkt 144–146 °C.

In analoger Weise erhält man:

| Bezeichnung | Ausbeute % d.Th. | Schmelzpunkt °C (Lösungsmittel) |
|---|---|---|
| a) 4-⟨2-Hydroxy-3-[4-(2-methoxy-phenoxymethyl)-piperidino]-propoxy⟩-2-benzimidazo- linon-hydrochlorid aus 2,3-Diamino-1-⟨2-hydroxy-3-[4-(2-methoxy-phenoxymethyl)-piperidino]-propoxy⟩-benzol-trihydrochlorid und Phosgen | 42 | 162–164 (Methanol) |
| b) 4-⟨2-Hydroxy-3-[4-(3-methyl-phenoxymethyl)-piperidino]-propoxy⟩-2-benzimidazolinon-hydrochlorid aus 2,3-Diamino-1-⟨2-hydroxy-3-[4-(3-methyl-phenoxymethyl)-piperidino]-propoxy⟩-benzol-trihydrochlorid und Phosgen | 40 | 227–229 (Methanol) |
| c) 4-[3-(4-Phenoxymethyl-piperidino)-propoxy]-2-benzimidazolinon-hydrochlorid aus 2,3-Diamino-1-[3-(4-phenoxymethyl-piperidino)-propoxy]-benzol-trihydrochlorid und Phosgen | 48 | 257–258 (Methanol) |
| d) 4-[2-Hydroxy-3-(4-phenoxymethyl-piperidino)-propoxy]-6-methyl-2-benzimidazolinon-hydrochlorid aus 2,3-Diamino-1-[2-hydroxy-3-(4-phenoxymethyl-piperidino)-propoxy]-5-methylbenzol-trihydrochlorid und Phosgen | 31 | 245–247 (Ethanol) |
| e) 4-[2-Hydroxy-3-(4-phenoxymethyl-piperidino)-propoxy]-6-tert.-butyl-2-benzimidazolinon-hydrochlorid aus 2,3-Diamino-1-[2-hydroxy-3-(4-phenoxymethyl-piperidino)-propoxy]-5-tert.-butyl-benzol-trihydrochlorid und Phosgen | 18 | 261–262 (Aceton) |
| f) 4-⟨2-Hydroxy-3-[4-(2-pyridyloxy-methyl)-piperidino]-propoxy⟩-2-benzimidazolinon-hydrochlorid aus 2,3-Diamino-1-⟨2-hydroxy-3-[4-(2-pyridyloxymethyl)-piperidino]-propoxy⟩-benzol-trihydrochlorid und Phosgen | 62 | 195–197 (Ethanol/ Methanol) |
| g) 4-⟨2-Hydroxy-3-[4-(4-2-benz-imidazolinonyloxymethyl)-piperidino]-propoxy⟩-2-benzimidazolinon-hydrochlorid aus 2,3-Diamino-1-⟨2-hydroxy-3-[4-(4-2-benzimidazolinonyloxymethyl)- | 20 | 223–226 (Methanol/ Wasser) |

| Bezeichnung | Ausbeute % d.Th. | Schmelzpunkt °C (Lösungsmittel) |
|---|---|---|
| piperidino]-propoxy⟩-benzol-tri-hydrochlorid und Phosgen | | |
| h) 4-⟨2-Hydroxy-3-[4-(4-methyl-(2)-pyrimidyloxymethyl)-piperidino]-propoxy⟩-2-benzimidazolinon-hydrochlorid aus 2,3-Diamino-1-⟨2-Hydroxy-3-[4-(4-methyl-(2)-pyrimidyloxymethyl)-piperidino]-propoxy⟩-benzoltrihydrochlorid und Phosgen | 25 | 152–155 (Ethanol/ Methanol |

Beispiel 2

4-[2-Pivaloyloxy-3-(4-phenoxymethyl-piperidino)-propoxy]-2-benzimidazolinon-hydrochlorid

5,00 g 4-[2-Hydroxy-3-(4-phenoxymethyl-piperidino)-propoxy 2-benzimidazolinon (Herstellung siehe Beispiel 1) werden zu 31,5 g geschmolzener Pivalinsäure gegeben und mit 6,28 g Pivalinsäureanhydrid versetzt. Man rührt 5 Tage bei Raumtemperatur, giesst in 100 ml Eiswasser, neutralisiert mit verdünntem Ammoniak (1:10), extrahiert mit Dichlormethan, trocknet über Natriumsulfat und engt ein. Der Rückstand wird mit Ether gewaschen, in Alkohol gelöst und mit 2 N Salzsäure versetzt.

Nach dem Einengen wird aus 20 ml Ethanol umkristallisiert. Man erhält 3,65 g (56% d.Th.) der Titelverbindung vom Schmelzpunkt 168–170 °C.

Beispiel 3

4-[2-Hydroxy-3-(4-phenoxymethyl-piperidino)-propoxy]-6,7-dimethyl-2-benzimidazolinon-hydrochlorid

Analog Beispiel 1 wird 2,3-Diamino-4,5-dimethyl-1-[2-hydroxy-3-(4-phenoxymethyl-piperidino)-propoxy]-benzol-trihydrochlorid mit Phosgen umgesetzt. Man erhält die Titelverbindung in 24% Ausbeute vom Schmelzpunkt 273–275 °C.

Das als Ausgangsmaterial verwendete 2,3-Diamino-4,5-dimethyl-1-[2-hydroxy-3-(4-phenoxymethyl-piperidino)-propoxy]-benzol-trihydrochlorid kann wie folgt hergestellt werden:

15,9 g 2-Amino-1-(2,3-epoxy-propoxy)-4,5-dimethyl-3-nitrobenzol und 12,8 g 4-Phenoxymethylpiperidin werden in 300 ml Ethanol 1,5 Stunden unter Rückfluss erhitzt. Die erkaltete Lösung wird zu 1,0 g Platinoxid in 100 ml Ethanol gegeben und bei Raumtemperatur hydriert. Nach Filtration säuert man mit verd. Salzsäure an und engt ein. Man nimmt den Rückstand in Ethanol/Wasser auf, behandelt mit Aktivkohle und erhält nach Einengen 28,4 g (84% d.Th.) vom Schmelzpunkt 144–148 °C.

In analoger Weise erhält man

a) 4-⟨2-Hydroxy-3-[4-(4-carboxamido-phenoxymethyl)piperidino]-propoxy⟩-6,7-dimethyl-2-benzimidazolinon-hydrochlorid mit Fp. 311–313 °C (Ethanol/Wasser) in 29% Ausbeute aus 2,3-Diamino-2-⟨2-hydroxy-3-[4-(4-carboxamido-phenoxymethyl)piperidino]-propoxy⟩-4,5-dimethylbenzol-trihydrochlorid und Phosgen.

Beispiel 4

Analog zu Beispiel 1 erhält man:

| Bezeichnung | Ausbeute % d.Th. | Schmelzpunkt °C (Lösungsmittel) |
|---|---|---|
| a) 4-[2-Hydroxy-3-(4-phenoxymethyl-piperidino)-propoxy]-7-methyl-2-benzimidazolinon-hydrochlorid aus 2,3-Diamino-1-[2-hydroxy-3-(4-phenoxymethyl-piperidino)-propoxy]-4-methyl-benzol-trihydrochlorid und Phosgen | 41 | 272–274 (Methanol) |
| b) 4-⟨2-Hydroxy-3-[4-(2-methoxy-phenoxymethyl)-piperidino]-propoxy⟩-7-methyl-2-benzimidazolinon-hydrochlorid aus 2,3-Diamino-1-⟨2-hydroxy-3-[4-(2- | 13 | 139–141 (Ethanol/ |

| Bezeichnung | Ausbeute % d.Th. | Schmelzpunkt °C (Lösungsmittel) |
|---|---|---|
| methoxy-phenoxymethyl)-piperidino]-propoxy 〉-4-methyl-benzol-tri-hydrochlorid und Phosgen | | Essigester) |
| c) 4-〈2-Hydroxy-3-[-4-(2-methoxy-4-methyl-phenoxymethyl)-piperidino]-propoxy〉 -7-methyl-2-benzimidazolinon-hydrochlorid aus 2,3-Diamino-〈2-hydroxy-3-[4-(2-methoxy-4-methyl-phenoxymethyl)-piperidino]-propoxy〉-4-methyl-benzol-trihydrochlorid und Phosgen | 17 | 248–250 (Isopropanol / Ethanol) |
| d) 4-〈2-Hydroxy-3-[4-(4-carboxamido-phenoxymethyl)-piperidino]-propoxy〉-7-methyl-2-benzimidazolinon-hydrochlorid aus 2,3-Diamino-1-〈2-hydroxy-3-[4-(4-carboxamido-phenoxymethyl)piperidino] propoxy〉-4-methyl-benzol-trihydrochlorid (amorph) und Phosgen | 37 | 294–296 (Methanol/ Wasser) |

Das für die Verbindung gemäss Beispiel 4 a) als Ausgangsmaterial benötigte 2,3-Diamino-1-[2-hydroxy-3-(4-phenoxymethyl-piperidino)-propoxy]-4-methyl-benzol-trihydrochlorid wird auf folgendem Weg erhalten:

84,0 g 1-(2,3-Epoxy-propoxy)-4-methyl-2,3-dinitro-benzol und 63,2 g 4-Phenoxymethylpiperidin werden in 500 ml Ethanol 2,5 Stunden unter Rückfluss gekocht. Der zur Trockene eingeengte Rückstand wird in 400 ml siedendem Essigester aufgenommen, mit Aktivkohle geklärt und nach Filtration mit 700 ml siedendem Ligroin versetzt. Man isoliert 103 g (68% d.Th.) 1-[2-Hydroxy-3-(4-phenoxymethyl-piperidino)-propoxy]-4-methyl-2,3-dinitrobenzol vom Schmelzpunkt 127–130 °C.

73,2 g dieser Dinitroverbindung, suspendiert in 500 ml Ethanol, werden zu 1,0 g Platinoxid in 150 ml Ethanol gegeben und bei Normaldruck und Raumtemperatur hydriert. Die Suspension wird in der Hitze gelöst, filtriert und eingeengt. Man nimmt den Rückstand in 500 ml heissem Ethanol auf, säuert mit konz. Salzsäure an, klärt mit Aktivkohle und erhält nach Einengen 70 g (89% d.Th.) amorphes Trihydrochlorid.

Analog werden durch Umsetzung eines entsprechend substituierten Piperidins mit 1-(2,3-Epoxy-propoxy)-4-methyl-2,3-dinitro-benzol und anschliessende Hydrierung die Ausgangssubstanzen für die Verbindungen gemäss der Beispiele 4 b), 4 c) und 4 d) erhalten:

| | Dinitroverbindung | Schmelzpunkt °C | o-Phenylendiamin-Derivat | Schmelzpunkt °C |
|---|---|---|---|---|
| für 4b) | 1-〈2-Hydroxy-3-[4-(2-methoxy-phenoxymethyl)-piperidino]-propoxy〉-4-methyl-2,3-dinitro-benzol | 138–140 | 2,3-Diamino-1〈2-hydroxy-3-[4-(2-methoxy-phenoxymethyl)-piperidino]-propoxy〉-benzol-trihydrochlorid | amorph |
| für 4c) | 1-〈2-Hydroxy-3-[4-(2-methoxy-4-methyl-phenoxymethyl)-piperidino]-propoxy〉-4-methyl-2,3-dinitrobenzol | nicht isoliert | 2,3-Diamino-1-〈2-hydroxy-3-[4-(2-methoxy-4-methyl-phenoxymethyl)-piperidino]-propoxy〉-4-methylbenzoltrihydrochlorid | amorph |
| für 4d) | 1-〈2-Hydroxy-3-[4-(4-carboxamido-phenoxymethyl)piperidino] propoxy〉-4-methyl-2,3-dinitrobenzol | 191–193 | 2,3-Diamino-1-〈2-Hydroxy-3-[4-(4-carboxamido-phenoxymethyl)piperidino]propoxy〉-4-methyl-benzol-trihydrochlorid | amorph |

Beispiel 5

4-⟨2-Hydroxy-3-[4-(2-methoxy-phenoxymethyl)-piperidino]-propoxy⟩-2-benzimidazolinthion-hydrochlorid

6,1 g 2,3-Diamino-1-⟨2-hydroxy-3-[4-(2-methoxy-phenoxymethyl)-piperidino]-propoxy⟩-benzol-trihydrochlorid wird mit 12,0 ml 1 M Natriummethylat-Lösung versetzt und zur Trockene eingeengt. Der Rückstand wird in 25 ml Ethanol gelöst und mit 2,1 g Kaliumxanthogenat 6 Stunden unter Stickstoff gekocht. Es wird heiss filtriert, das Filtrat mit Aktivkohle geklärt und vollständig eingeengt. Nach Umkristallisation aus Ethanol erhält man 1,26 g (22% d.Th.) Titelverbindung vom Schmelzpunkt 212–215 °C.

Beispiel 6

4-⟨2-Hydroxy-3-[4-(3-methyl-phenoxymethyl)-piperidino]-propoxy⟩-2-benzimidazolinthion-hydrochlorid

(Schmelzpunkt 236–237 °C aus Methanol) wird analog Beispiel 5 aus 2,3-Diamino-1-⟨2-hydroxy-3-[4-(3-methyl-phenoxymethyl)-piperidino]-propoxy⟩-benzol und Kaliumxanthogenat erhalten.

Beispiel 7

4-[2-Hydroxy-3-(4-phenoxymethyl-piperidino)-propoxy]-7-methyl-3-propyl-2-benzimidazolinon

Analog Beispiel 1 wird aus 3-Amino-1-[2-hydroxy-3-(4-phenoxymethyl-piperidino)-propoxy]-4-methyl-2-propylamino-benzol-trihydrochlorid und Phosgen mit 54% Ausbeute die Titelverbindung vom Schmelzpunkt 234–235 °C erhalten.

Das hierzu benötigte Ausgangsmaterial wird wie folgt hergestellt:

15,0 g 1-[2-Hydroxy-3-(4-phenoxymethyl-piperidino)-propoxy]-4-methyl-2,3-dinitro-benzol (siehe Herstellung des Ausgangsproduktes zu Beispiel 4a)) wird in 12 ml n-Propylamin 3,5 Stunden zum Rückfluss erhitzt. Nach Abkühlen werden 8,8 g (59% d.Th.) kristallines 1-[2-Hydroxy-3-(4- phenoxymethyl-piperidino)-propoxy]-4-methyl-2-propylamino-3-nitro-benzol vom Schmelzpunkt 91–93 °C isoliert.

Diese Verbindung wird über Platinoxid zum gewünschten 3-Amino-1-[2-hydroxy-3-(4-phenoxymethyl-piperidino)-propoxy]-4-methyl-2-propylamino-benzol hydriert.

Beispiel 8

4-[2-Hydroxy-3-(4-phenoxymethyl-piperidino)-propoxy]-oxindol-acetat

Eine Lösung von 7,8 g 2-[2-Hydroxy-3-(4-phenoxymethyl-piperidino)-propoxy]-6-nitro-phenylessigsäureethylester in 100 ml Methanol und 100 ml Essigsäure wird bei Raumtemperatur und 1 bar Wasserstoffdruck über 10%ige Palladiumkohle hydriert. Nach Absaugen des Katalysators wird im Vakuum abdestilliert und der verbleibende Rückstand in Wasser gelöst und filtriert. Durch Zugabe von Natriumcarbonatlösung wird die Base ausgefällt und abgesaugt. Durch Auflösen der Base in etwa 150 ml Essigester und 5 ml Essigsäure in der Siedehitze erhält man nach Abkühlen und Absaugen 2,1 g 4-[2-Hydroxy-3-(4-phenoxymethyl-piperidino)-propoxy]-oxindolacetat (27% d.Th.) vom Schmelzpunkt 125–130 °C.

In analoger Weise wie in Beispiel 8 beschrieben erhält man:

| Bezeichnung | Ausbeute % d.Th. | Schmelzpunkt °C (Lösungsmittel) |
|---|---|---|
| a) 4-⟨2-Hydroxy-3-[4-(2-methoxy-phenoxymethyl)-piperidino]-propoxy⟩-oxindol-acetat aus 2-⟨2-Hydroxy-3-[4-(2-methoxy-phenoxymethyl)-piperidino]-propoxy⟩-6-nitro-phenylessigsäureethylester | 37 | 114–118 (Essigester) |
| b) 4-⟨2-Hydroxy-3-[4-(3-methyl-phenoxymethyl)-piperidino]-propoxy⟩-oxindol-acetat aus 2-⟨2-Hydroxy-3-[4-(3-methyl-phenoxymethyl)-piperidino]-propoxy⟩-6-nitro-phenylessigsäureethylester | 28 | 161–162 (Essigester) |
| c) 4-⟨2-Hydroxy-3-[4-(4-hydroxy-phenoxymethyl)-piperidino]-propoxy⟩-oxindol aus 2-⟨2-Hydroxy-3-[4-(4-benzyloxy-phenoxymethyl)-piperidino]-propoxy⟩-6-nitro-phenylessgisäureethylester | 38 | 192 (Ethanol) |

| Bezeichnung | Ausbeute % | Schmelzpunkt °C (Lösungsmittel) |
|---|---|---|
| d) 4-⟨2-Hydroxy-3-[4-(2-chlor-phenoxy-methyl)-piperidino]-propoxy⟩-oxindol aus 2-⟨ 2-Hydroxy-3-[4-(2-chlor-phenoxy-methyl)-piperidino]-propoxy⟩-6-nitro-phenylessigsäureethylester | 33 | 164–165 (Methanol) |
| e) 4-⟨ 2-Hydroxy-3-[4-(3-hydroxy-methyl-phenoxymethyl)-piperidino]-propoxy⟩ -oxindol aus 2-⟨ 2-Hydroxy-3-[4-(3-hydroxy-methyl-phenoxymethyl)-piperidino]-propoxy⟩ -6-nitro-phenylessigsäure-ethylester | 27 | 152–154 (Methanol) |
| f) 4- ⟨ 2-Hydroxy-3-[4-(2-methyl-6-pyridoxymethyl)-piperidino]-propoxy⟩-oxindol aus 2-⟨ 2-Hydroxy-3-[4-(2-methyl-6-pyridoxymethyl)-piperidino]-propoxy⟩ -6-nitro-phenylessigsäure-ethylester | 17 | 165–167 (Methanol) |
| g) 4-⟨ 2-Hydroxy-3-[4-(4-aminocarbonyl-methyl-phenoxymethyl)-piperidino]-propoxy⟩ -oxindol-acetat aus 2- ⟨ 2-Hydroxy-3-[4-(4-aminocarbonyl-methyl-phenoxymethyl)-piperidino]-propoxy ⟩ -6-nitro-phenylessigsäure-ethylester | 46 | 173–175 (Methanol) |

Der zur Herstellung der vorstehenden Verbindung des Beispiels 8 als Ausgangsstoff benötigte 2-[2-Hydroxy-3-(4-phenoxymethyl-piperidino)-propoxy]-6-nitro-phenylessigsäureethylester wird wie folgt erhalten:

2-Allyloxy-6-nitro-toluol

76,6 g 2-Methyl-3-nitro-phenol werden in 200 ml Methanol mit 84,6 ml Allylbromid versetzt und 375 ml 2 N Natriummethylatlösung zugetropft. Nach 18 Stunden Rühren bei Raumtemperatur wird abdestilliert und der Rückstand in Wasser und Ether aufgenommen. Nach Eindampfen der Etherphase werden 95,7 g 2-Allyloxy-6-nitro-toluol erhalten (99% d.Th.).

2-Allyloxy-6-nitro-phenylbrenztraubensäureethylester

0,625 mol Kalium-tert.-butylat, 542 ml Oxalsäurediethylester und 95,7 g 2-Allyloxy-6-nitro-toluol lässt man 3 Stunden bei 60 °C rühren, versetzt mit 1 N Essigsäure und extrahiert mit Ether. Den im Etherrückstand verbleibenden Oxalester entfernt man bei erhöhter Temperatur im Wasserstrahlvakuum. Es werden 171 g 2-Allyloxy-6-nitro-phenylbrenztraubensäureethylester erhalten.

2-Allyloxy-6-nitro-phenylessigsäure

171 g 2-Allyloxy-6-nitro-phenylbrenztraubensäureethylester werden in 1100 ml 1 N Natronlauge mit 6%igem Wasserstoffperoxyd oxidiert und die erhaltene Säure durch Lösen in Natriumhydrogencarbonat, Filtrieren und Ausfällen mit Salzsäure gereinigt. Nach Trocknen verbleiben 88,5 g 2-Allyloxy-6-nitro-phenylessigsäure vom Schmelzpunkt 115–117 °C (74% d.Th., bezogen auf 2-Allyloxy-6-nitro-toluol).

2-Allyloxy-6-nitro-phenylessigsäureethylester

23,7 g 2-Allyloxy-6-nitro-phenylessigsäure werden mit 17,4 ml Ethanol in Toluol und 1,9 g p-Toluolsulfonsäure in den gewünschten Ester überführt. Nach Aufarbeiten werden 25,9 g 2-Allyloxy-6-nitro-phenylessigsäureethylester erhalten (97% d.Th.).

2-(2,3-Epoxy-propoxy)-6-nitro-phenylessigsäureethylester

10,6 g 2-Allyloxy-6-nitro-phenylessigsäureethylester in 250 ml Chloroform werden bis zur völligen Umsetzung mit 14,6 g m-Chlor-peroxybenzoesäure zum Sieden erhitzt. Nach der üblichen Aufarbeitung werden 11 g 2-(2,3-Epoxy-propoxy)-6-nitro-

phenylessigsäureethylester erhalten (99% d.Th.).

2-[-2-Hydroxy-3-(4-phenoxymethyl-piperidino)-propoxy]-6-nitro-phenylessigsäureethylester

6,5 g 2-(2,3-Epoxy-propoxy)-6-nitro-phenylessigsäureethylester und 4,4 g 4-Phenoxymethylpiperidin werden in 65 ml n-Butanol 18 Stunden bei Raumtemperatur gerührt, im Vakuum eingeengt und in Ether und 1 N Milchsäure aufgenommen. Die wässrige Phase wird mit Kaliumcarbonatlösung alkalisch gestellt und mit Ether extrahiert. Nach Trocknen und Eindampfen verbleiben 7,9 g 2-[2-Hydroxy-3-(4-phenoxymethyl-piperidino)-propoxy]-6-nitro-phenylessigsäure als bräunliches Öl (72% d.Th.).

In analoger Weise werden die Ausgangssubstanzen für die Verbindungen der Beispiele 8 a) – 8 g) erhalten.

| | Bezeichnung | Ausbeute % | Schmelzpunkt °C (Lösungsmittel) |
|---|---|---|---|
| für 8a) | 2-⟨ 2-Hydroxy-3-[4-(2-methoxy-phenoxymethyl)-piperidino]-propoxy⟩-6-nitro-phenylessigsäureethylester aus 2-(2,3-Epoxy-propoxy)-6-nitrophenyl-essigsäureethylester und 4-(2-Methoxy-phenoxymethyl)-piperidin | 72 | Öl |
| für 8b) | 2-⟨ 2-Hydroxy-3-[4-(3-methyl-phenoxy-methyl)-piperidino]-propoxy⟩-6-nitro-phenylessigsäureethylester aus 2-(2,3-Epoxy-propoxy)-6-nitro-phenyl-essigsäureethylester und 4-(3-Methyl-phenoxymethyl)-piperidin | 79 | Öl |
| für 8c) | 2-⟨ 2-Hydroxy-3-[4-(4-benzyloxy-phenoxymethyl)-piperidino]propoxy⟩-6-nitro-phenylessigsäureethylester aus 2-(2,3- Epoxy-propoxy)-6-nitro-phenyl-essigsäureethylester und 4-(4-Benzyloxy-phenoxymethyl)-piperidin | 87 | Öl |
| für 8d) | 2- ⟨ 2-Hydroxy-3-[-4-(2-chlor-phenoxy-methyl)-piperidino]-propoxy⟩-6-nitro-phenylessigsäureethylester aus 2-(2,3-Epoxy-propoxy)-6-nitro-phenylessigsäureethylester und 4-(2-Chlor-phenoxymethyl)-piperidin | 65 | Öl |
| für 8e) | 2- ⟨ 2-Hydroxy-3-[4-(3-hydroxymethyl-phenoxymethyl)-piperidino]-propoxy⟩ -6-nitro-phenylessigsäure-ethylester aus 2-(2,3-Epoxy-propoxy)-6-nitro-phenylessigsäureethylester und 4-(3-Hydroxymethyl-phenoxymethyl)-piperidin | 80 | Öl |
| für 8f) | 2- ⟨ 2-Hydroxy-3-[4-(2-methyl-6-pyridoxymethyl)-piperidino]-propoxy⟩ -6-nitro-phenylessigsäure-ethylester aus 2-(2,3-Epoxy-propoxy)-6-nitro- | 99 | Öl |

| | Bezeichnung | Ausbeute % | Schmelzpunkt °C (Lösungsmittel) |
|---|---|---|---|
| | phenylessigsäureethylester und 4-(2-Methyl-6-pyridoxymethyl)-piperidin | | |
| für 8g) | 2-⟨2-Hydroxy-3-[4-(4-aminocarbonyl-methyl-phenoxymethyl)-piperidino]-propoxy⟩-6-nitro-phenylessigsäure-ethylester aus 2-(2,3-Epoxy-propoxy)-6-nitro-phenyl-essigsäureethylester und 4-(4-Aminocarbonylmethyl-phenoxymethyl)-piperidin | 95 | Öl |

Beispiel 9

4-⟨2-Hydroxy-3-[4-(2-pyridyloxymethyl)-piperidino]-propoxy⟩-indazol

Ein Gemisch aus 5,6 g 2-Benzyl-4-(2,3-epoxy-propoxy)-indazol, 3,9 g 4-(2-Pyridyloxymethyl)-piperidin und 5 ml 1,2-Dimethoxyethan wird 20 Stunden auf 70 °C erwärmt. Anschliessend engt man ein, nimmt den Rückstand in 120 ml Methanol auf, fügt 10 ml konz. Salzsäure zu und hydriert in Gegenwart von Palladium-Kohle (10%) bei Normaldruck. Nach Absaugen des Katalysators engt man ein, löst in Wasser, stellt mit Natronlauge alkalisch und nimmt in Dichlormethan auf. Das nach Trocknen mit Natriumsulfat und Einengen erhaltene Öl reibt man mit Ether an und erhält nach Umkristallisation aus Essigester unter Verwendung von Aktivkohle 3,9 g (51% d.Th.) Titelverbindung vom Schmelzpunkt 81–83 °C.

Das als Ausgangsstoff verwendete 2-Benzyl-4-(2,3-epoxy-propoxy)-indazol kann wie folgt erhalten werden:

Man trägt unter Kühlen und Stickstoffatmosphäre 9,2 g Natriumhydrid (55–60% Suspension in Paraffin) in eine Lösung von 47,1 g 2-Benzyl-4-hydroxy-indazol in 250 ml Dimethylformamid ein, tropft nach beendeter Wasserstoffentwicklung 19 ml Epibromhydrin zu und rührt 16 Stunden bei Raumtemperatur. Es wird in 1,5 Liter Wasser eingegossen, mit Dichlormethan extrahiert und das erhaltene Öl über eine Kieselgelsäule (Laufmittel: Dichlormethan/Methanol 99:1) gereinigt. Aus dem Rückstand der Anfangsfraktionen erhält man durch Anreiben mit Ligroin-Ether (1:1) 30,0 g (51% d.Th.) farblose Kristalle vom Schmelzpunkt 66–68 °C.

In analoger Weise erhält man durch Umsetzen von 2-Benzyl-4-(2,3-epoxy-propoxy)-indazol mit 4-(4-Benzimidazolinonyl-oxymethyl)-piperidin und anschliessender Hydrierung in 44% Ausbeute 4-⟨2-Hydroxy-3-[4-2-benzimidazolinonyloxymethyl)-piperidino]-propoxy⟩-indazol vom Schmelzpunkt 253–255 °C.

Beispiel 10

4-[2-Hydroxy-3-(4-phenoxymethyl-piperidino)-propoxy]-carbazol-hydrochlorid

4,3 g 4-Phenoxymethylpiperidin und 5,35 g 4-(2,3-Epoxy-propoxy)-carbazol werden 4 Stunden auf 120 °C erhitzt. Die kalte Reaktionsmischung wird in Aceton gelöst und mit etherischer Salzsäure versetzt. Man saugt das ausgefallene Hydrochlorid ab, kristallisiert aus Isopropanol/Ethanol um und erhält 8,0 g (69% d.Th.) Titelverbindung vom Schmelzpunkt 224–225 °C.

In analoger Weise werden aus 4-(2,3-Epoxy-propoxy)-carbazol und dem entsprechend substituierten 4-Methylpiperidin-Derivat erhalten:

| Bezeichnung | Ausbeute % d.Th. | Schmelzpunkt °C (Lösungsmittel) |
|---|---|---|
| a) 4-⟨2-Hydroxy-3-[4-(2-chlor-phenoxymethyl)-piperidino]-propoxy⟩-carbazol-hydrochlorid | 61 | 213–215 (Ethanol/Methanol) |
| b) 4-⟨2-Hydroxy-3-[4-2-methoxy-phenoxymethyl)-piperidino]-propoxy⟩-carbazol-hydrochlorid | 65 | 180 (Zers.) (Ethanol/Methanol) |
| c) 4-⟨2-Hydroxy-3-[4-(2-methyl-phenoxymethyl)-piperidino]-propoxy⟩-carbazol-hydrochlorid | 83 | 150 (Zers.) (Aceton) |

| Bezeichnung | Ausbeute % d.Th. | Schmelzpunkt °C (Lösungsmittel) |
|---|---|---|
| d) 4-⟨2-Hydroxy-3-[4-(3-methyl-phenoxymethyl)-piperidino]-propoxy⟩-carbazol-hydrochlorid | 74 | 212–215 (Aceton) |
| e) 4-⟨2-Hydroxy-3-[4-(2-pyridyloxymethyl)-piperidino]-propoxy⟩-carbazol-hydrochlorid | 50 | 180–182 (Ethanol) |

Beispiel 11

4-⟨2-Hydroxy-3-[4-(4-2-benzimidazolinonyl-oxy-methyl)-piperidino]-propoxy⟩-carbazol-hydrochlorid

3,6 g 4-(2,3-Epoxy-propoxy)-carbazol und 3,7 g 4-(4-2-benzimidazolinonyloxymethyl)-piperidin werden in 75 ml Ethanol und 10 ml Wasser 20 Stunden unter Rückfluss erhitzt. Der eingeengte Rückstand wird über eine Kieselgelsäule (Laufmittel: Dichlormethan/Methanol 80:20) chromatografiert. Man nimmt in Tetrahydrofuran auf und fällt mit Tetrahydrofuran/Salzsäure. Ausbeute: 3,1 g (43% d.Th.) der Titelverbindung als Hydrochlorid vom Schmelzpunkt 185 °C (Zers.).

Beispiel 12

4-⟨2-Hydroxy-3-[4-(4-2-benzimidazolinonyl-oxy-methyl)-piperidino]-propoxy⟩-6-methyl-benzotriazol-hydrochlorid

7,7 g 2,3-Diamino-1-⟨2-hydroxy-3-[4-(4-2-benzimidazolinonyloxymethyl)-piperidino]-propoxy⟩-5-methyl-benzol-trihydrochlorid löst man in 45 ml Wasser und 17 ml Eisessig. Die auf 0 °C gekühlte Lösung wird mit 1,0 g Natriumnitrit in 1,6 ml Wasser versetzt und 1 Stunde bei Raumtemperatur nachgerührt. Die feste Abscheidung wird isoliert und aus Ethanol/Essigester umkristallisiert. Man erhält 3,4 g (50% d.Th.) Titelverbindung vom Schmelzpunkt 227–230 °C.

Die als Ausgangsmaterial eingesetzte Diaminoverbindung wird wie folgt erhalten:

6,72 g 2-(2,3-Epoxy-propoxy)-4-methyl-6-nitroanilin und 7,10 g 4-2-Benzimidazolinonyloxymethylpiperidin werden in 200 ml Ethanol 8 Stunden unter Rückfluss gekocht. Die Lösung wird zu 0,3 g Platindioxid in 100 ml Ethanol zugegeben und bei Raumtemperatur und Normaldruck hydriert. Man filtriert, säuert mit etherischer Salzsäure an und saugt 7,7 g 2,3-Diamino-1-⟨2-hydroxy-3-[4-(4-2-benzimidazolinonyl-oxymethyl)-piperidino]-propoxy⟩-5-methyl-benzol-trihydrochlorid ab.

Beispiel 13

4-⟨2-Hydroxy-3-[4-(2-pyridyloxymethyl)-piperidino]-propoxy⟩-7-methyl-benzimidazol-hydrochlorid

18,0 g 2,3-Diamino-1-⟨2-hydroxy-3-[4-(2-pyridyloxymethyl)-piperidino]-propoxy⟩-4-methylbenzol-trihydrochlorid wird in 60 ml Ameisensäure 2 Stunden rückfliessend gekocht. Man destilliert die Ameisensäure vollständig ab, kocht 2 Stunden in 60 ml 2 N Salzsäure, klärt mit Aktivkohle und engt zur Trockene ein. Der Rückstand wird aus 160 ml Ethanol/40 ml Methanol kristallisiert. Man erhält 8,5 g (54% d.Th.) der Titelverbindung vom Schmelzpunkt 188–190 °C.

Beispiel 14

4-⟨2-Hydroxy-3-[4-(2-pyridyloxymethyl)-piperidino]-propoxy⟩-2-methyl-benzimidazol-hydrochlorid

In 50 ml Eisessig werden 9.6 g 2,3-Diamino-1-⟨2-hydroxy-3-[4-(2-pyridyloxymethyl)-piperidino]-propoxy⟩-benzol-trihydrochlorid 3 Stunden gekocht. Nach Einengen zur Trockene verseift man die teilweise entstandene 2-O-Acetylverbindung in 50 ml 2 N Salzsäure 2 Stunden unter Rückflusskochen. Nach Klären mit Aktivkohle wird eingeengt und aus Ethanol/Essigester kristallisiert. Man isoliert 2,3 g (27% d.Th.) Titelverbindung vom Schmelzpunkt 166–169 °C.

Beispiel 15

4-⟨2-Hydroxy-3-[4-(2-n-butoxy-phenoxymethyl)-piperidino]-propoxy⟩-1-formylindolin-p-chlorbenzoat

Eine Lösung von 2,2 g 4-(2,3-Epoxy-propoxy)-1-formylindolin und 2,6 g 4-(2-Butoxy-phenoxymethyl)-piperidin in 50 ml n-Butanol lässt man 18 Stunden rühren, engt ein, löst in Essigester und gibt die äquivalente Menge p-Chlorbenzoesäure zu. Nach Absaugen und Umkristallisieren aus Essigester erhält man 3,3 g (51% d.Th.) 4-⟨2-Hydroxy-3-[4-(2-n-butoxy-phenoxymethyl)-piperidino]-propoxy⟩-1-formylindolin-p-chlorbenzoat vom Schmelzpunkt 110–113 °C.

In analoger Weise erhält man

| Bezeichnung | Ausbeute % d.Th. | Schmelzpunkt °C (Lösungsmittel) |
|---|---|---|
| a) 4-⟨2-Hydroxy-3-[4-(3,4-methylendioxy-phenoxymethyl)-piperidino]-propoxy⟩-1-formylindolin aus 4-(2,3-Epoxy-propoxy)-1-formylindolin und 4-(3,4-Methylendioxy-phenoxymethyl)-piperidin | 59 | 130–132 (Isopropanol) |
| b) 4-⟨2-Hydroxy-3-[4-(4-amino-phenoxymethyl)-piperidino]-propoxy⟩-1-formylindolin aus 4-(2,3-Epoxy-propoxy)-1-formylindolin und 4-(4-Amino-phenoxymethyl)-piperidin | 25 | 75 |
| c) 4-⟨2-Hydroxy-3-[4-(4-acetamido-phenoxymethyl)-piperidino]-propoxy⟩-1-formylindolin aus 4-(2,3-Epoxy-propoxy)-1-formylindolin und 4-(4-Acetamido-phenoxymethyl)-piperidin | 67 | 178–180 (Butanol) |
| d) 4-⟨2-Hydroxy-3-[4-(4-methansulfonylamido-phenoxymethyl)-piperidino]-propoxy⟩-1-formylindolin aus 4-(2,3-Epoxy-propoxy)-1-formylindolin und 4-(4-Methansulfonylamido-phenoxymethyl)-piperidin | 25 | 127–128 (Methanol) |

Das zur Herstellung der vorstehenden Verbindungen als Ausgangsstoff benötigte 4-(2,3-Epoxy-propoxy)-1-formylindolin wird wie folgt hergestellt:

Zu 48,6 g 2-Benzyloxy-6-nitrotoluol und 29,9 g Paraformaldehyd gelöst in 670 ml Dimethylformamid werden 200 ml 1 N Kalium-tert.-butylatlösung zugetropft. Nach 1 Stunde Rühren bei Raumtemperatur wird in 3 Liter Eiswasser eingerührt und mit Ether extrahiert. Die Etherphase wird mit $Na_2SO_4$ getrocknet und im Vakuum eingeengt. Es verbleiben 62 g 2-Benzyloxy-6-nitrophenyl-ethanol, welches als Rohprodukt in die nächste Stufe eingesetzt wird.

62,0 g 2-Benzyloxy-6-nitro-phenylethanol werden in 500 ml wasserfreiem Pyridin gelöst und unter Kühlen bei ca. 10 °C mit 47,7 g p-Toluolsulfonylchlorid versetzt. Man lässt auf Raumtemperatur ansteigen und rührt bis zur völligen Umsetzung ca. 10 Stunden. Die Reaktionslösung wird in Eiswasser eingerührt. Nach Absaugen, Waschen mit Wasser und Trocknen verbleiben 74 g (86% d.Th.) p-Toluolsulfonsäure-2-(2-benzyloxy-6-nitrophenyl)-ethylester vom Schmelzpunkt 96–98 °C.

74 g p-Toluolsulfonsäure-2-(2-benzyloxy-6-nitrophenyl)-ethylester werden in 2 Liter Ethylenglykolmonomethylether gelöst, mit 5 g 10%iger Palladium-Aktivkohle versetzt und bei Raumtemperatur und 1 bar Wasserstoffdruck hydriert. Nach Entfernen des Katalysators wird eingeengt und der Rückstand mit einer Mischung aus 227 ml Essigsäureanhydrid und 91 ml Ameisensäure (nach C.W. Huffmann, J. org. Chem. 23, 727 [1958]) formyliert. Nach der Umsetzung wird mit Eiswasser zersetzt und mit Essigester extrahiert. Die organische Phase wird neutralisiert, über Natriumsulfat getrocknet und im Vakuum eingedampft. Den verbleibenden Rückstand vermischt man mit 320 ml Epichlorhydrin und versetzt mit 173 ml 2 N Natriumethylatlösung. Nach Rühren über Nacht wird die Reaktionsmischung eingeengt und der Rückstand in Wasser und Essigester gelöst. Aus dem Essigesterrückstand werden durch Anreiben mit Isopropanol und Absaugen 15,8 g (42% d.Th.) 4-(2,3-Epoxy-propoxy)-1-formylindolin vom Schmelzpunkt 88–89 °C erhalten.

Beispiel 16

4-⟨2-Hydroxy-3-[4-(4-acetamido-phenoxymethyl)-piperidino]-propoxy⟩-1-formylindolin

3,3 g 4-⟨2-Hydroxy-3-[4-(4-amino-phenoxymethyl)-piperidino]-propoxy⟩-1-formylindolin (Herstellung siehe Beispiel 15 b)) werden mit einer Mischung von 25 ml Essigsäureanhydrid und

25 ml Pyridin 10 Stunden bei Raumtemperatur gerührt, im Vakuum eingeengt und in Wasser und Dichlormethan gelöst. Nach Neutralisieren mit Natriumhydrogencarbonat wird die organische Phase abdestilliert und der verbleibende Rückstand in Methanol mit Natriummethylatlösung in die gewünschte Verbindung überführt. Durch Ausschütteln zwischen Dichlormethan und Wasser und Eindampfen der organischen Phase erhält man 1,0 g (27% d.Th.) 4-⟨-2-Hydroxy-3-[4-(4-acetamido-phenoxy-methyl)-piperidino]-propoxy⟩-1-formylindolin vom Schmelzpunkt 177–179 °C.

In analoger Weise erhält man:

| Bezeichnung | Ausbeute % d.Th. | Schmelzpunkt °C (Lösungsmittel) |
|---|---|---|
| a) 4-⟨2-Hydroxy-3-[4-(4-methansulfonyl-amido-phenoxymethyl)-piperidino]-propoxy⟩ -1-formylindolin aus 4-⟨2-Hydroxy-3-[4-(4-amino-phenoxy-methyl)-piperidino]-propoxy⟩ -1-formylindolin und Methansulfonsäurechlorid | | |

Beispiel 17

4-[3-(4-Phenoxymethyl-piperidino)-propoxy]-carbazol

Zu der Lösung von 0,55 g Natrium in 100 ml Isopropanol gibt man 4,6 g 4-Hydroxy-carbazol, erwärmt 10 min zum Rückfluss, fügt bei Raumtemperatur 6,9 g 3-(4-Phenoxymethyl-piperidino)-propylchlorid (DE-OS 25 50 000) zu, erwärmt 5 Stunden zum Rückfluss, engt ein, nimmt den Rückstand in verd. Natronlauge auf, extrahiert mit Ether und engt den Extrakt ein. Nach Umkristallisation aus Isopropanol/Ligroin verbleiben 4,7 g Titelverbindung (46% d.Th.) vom Schmelzpunkt 126–128 °C.

Beispiel 18

In analoger Weise, wie in Beispiel 15 beschrieben, erhält man:

| Bezeichnung | Ausbeute % d.Th. | Schmelzpunkt °C (Lösungsmittel) |
|---|---|---|
| a) 4-⟨ 2-Hydroxy-3-[4-(2-pyridyloxymethyl) piperidino]propoxy⟩-indol-benzoat aus 4-(2,3-Epoxy-propoxy)indol und 4-(2-Pyridyloxymethyl)piperidin | 45 | 117–119 (Isopropanol) |
| b) 4-⟨ 2-Hydroxy-3-[4-(3,4-methylendioxy-phenoxymethyl)-piperidino]propoxy⟩ indol-benzoat aus 4-(2,3-Epoxy-propoxy)-indol und 4-(3,4-Methylendioxy-phenoxymethyl)-piperidin | 72 | 148–150 (Essigester) |
| c) 4-⟨ 2-Hydroxy-3-[4-(4-2-benzimidazolinonyl-oxymethyl) piperidino]-propoxy⟩ indol-acetat aus 4-(2,3-Epoxy-propoxy)indol und 4-(4-2-Benzimidazolinonyloxymethyl)-piperidin | 52 | 208–210 (Butanol) |
| d) 4-⟨ 2-Hydroxy-3-[4-(4-2-benzimidazolinonyl-oxymethyl)-piperidino]propoxy⟩ -2-hydroxy-methylindol-acetat aus 4-(2,3-Epoxy-propoxy)-2-hydroxymethylindol | 58 | 147–178 (Ethanol) |

| Bezeichnung | Ausbeute % d.Th. | Schmelzpunkt °C (Lösungsmittel) |
|---|---|---|
| und 4-(4-2-Benzimidazolinonyloxymethyl)piperidin | | |
| e) 4-⟨2-Hydroxy-3-[4-(2-pyridyloxymethyl)piperidino]-propoxy⟩-2-hydroxymethylindol-benzoat aus 4-(2,3-Epoxy-propoxy)-2-hydroxymethylindol und 4-(2-Pyridyloxymethyl)piperidin | 55 | 76–78 (Isopropanol) |
| f) 4-⟨2-Hydroxy-3-[4-(2-pyridyloxymethyl)piperidino]-propoxy⟩-2-ethoxycarbonylindol aus 4-(2,3-Epoxy-propoxy)-2-ethoxycarbonylindol und 4-(2-Pyridyloxymethyl)-piperidin | 51 | 154–156 (Isopropanol) |

Beispiel 19

4-⟨2-Hydroxy-3-[4-(2-pyridyloxymethyl)piperidino]propoxy⟩ -2-hydroxymethylindol-benzoat

Zu einer Suspension von 1 g Lithiumaluminiumhydrid in 125 ml abs. Tetrahydrofuran tropft man eine Lösung von 4,6 g 4-⟨2-Hydroxy-3-[4-(2-pyridyloxymethyl)piperidino]propoxy⟩-2-ethoxycarbonylindol (Herstellung siehe Beispiel 18 f)) in 125 ml abs. Tetrahydrofuran, rührt 30 Minuten nach, zersetzt unter Kühlung mit Natriumchloridlösung und 10 N Natronlauge, filtriert, wäscht mit Tetrahydrofuran nach und engt ein. Durch Zugabe der äquivalenten Menge Benzoesäure erhält man aus Isopropanol 4,0 g (74% d.Th.) der Titelverbindung vom Schmelzpunkt 76–78 °C.

Beispiel 20

4-⟨2-Benzoyloxy-3-[4-(2-pyridyloxymethyl)piperidino]propoxy⟩-7-methyl-benzimidazol-hydrochlorid

4,87 g 4-⟨2-Hydroxy-3-[4-(2-pyridyloxymethyl)-piperidino]propoxy⟩ -7-methyl-benzimidazol (Herstellung siehe Beispiel 13), 19,5 g Benzösäure und 2,12 g Benzösäureanhydrid werden in 100 ml Benzol und 25 ml Dimethylformamid 2 Stunden unter Rückfluss erhitzt. Nach Entfernen des Lösungsmittels wird der Rückstand in 100 ml Wasser aufgenommen, mit konz. Ammoniak alkalisch gestellt und mit Chloroform ausgezogen. Die Chloroformphase wäscht man mit Wasser, trocknet über Natriumsulfat und engt ein. Es wird in Ethanol aufgenommen und mit etherischer Salzsäure versetzt. Nach Zusatz von Isopropanol und Ether kristallisieren 2,1 g (41% d.Th.) Titelverbindung vom Fp. 178–181 °C.

Beispiel 21

Es wurden Tabletten hergestellt: Jede Tablette enthält 10 mg 4-[2-Hydroxy-3-(4-phenoxymethylpiperidino)-propoxy]-2-benzimidazolinon. Die Tabletten wurden gemäss der folgenden Rezeptur hergestellt:

| | |
|---|---|
| 4-[2-Hydroxy-3-(4-phenoxymethylpiperidino)-propoxy]-2-benzimidazolinon | 10 g |
| Lactose | 80 g |
| Stärke | 29 g |
| Magnesiumstearat | 1 g |

Vorstehende Verbindung wurde fein pulverisiert und mit Lactose und Stärke vermischt. Das Gemisch wurde in herkömmlicher Weise granuliert. Magnesiumstearat wurde zu dem Granulat gegeben und das Gemisch zu 1000 Tabletten mit einem Einzelgewicht von 0,12 g verpresst.

Die erfindungsgemässen neuen Verbindungen wurden hinsichtlich ihrer vasodilatierenden und β-blockierenden Aktivität geprüft. Da beide Eigenschaften nicht in einem einzelnen Versuchsmodell untersucht werden können, mussten verschiedene Versuchsanordnungen gewählt werden.

a) Prüfung auf vasodilatierende Aktivität:

Die Vasodilatation macht sich durch Blutdrucksenkung bemerkbar. Kaninchen wurden mit Urethan narkotisiert. Zur fortlaufenden Messung des arteriellen Blutdruckes wurde ein Katheter in die A.femoralis implantiert. Die Messung des Blutdruckes erfolgte mittels eines elektromechanischen Druckwandlers (Statham P 23 Db). Die Impulse wurden auf einem Direktschreiber aufgezeichnet und nach Eichen mit einem Quecksilbermanometer ausgewertet.

Nach Ermittlung des Ausgangswertes wurden beide Halsschlagadern (A. carotis) für 2 min okkludiert und auf diese Weise der Blutdruck temporär erhöht (CSE-Reflex). Anschliessend wurde die Prüfsubstanz in der niedrigsten Prüfdosis (0,125 mg/kg) i.v. injiziert und weitere 8 min später wurden in logarithmisch steigender Dosierung (Faktor 2) die Prüfsubstanzen erneut injiziert und der CSE wiederum ausgelöst (Dosen: 0,125, 0,125, 0,25, 1,0 . . .mg/kg).

Substanzen, die unter diesen Bedingungen den Anstieg des Blutdruckes unter CSE abschwächen, können als vasodilatierend angesehen werden. Errechnet wurde von den Prüfsubstanzen die Dosis, die den CSE-Reflex um 30 mm Hg abschwächt ($ED_{-30mm\ Hg}$).

Da in jedem Versuch die Dosis solange erhöht wurde, bis das Tier an toxischen Erscheinungen starb, konnte für jedes Tier die tödliche Dosis bestimmt werden (letale Dosis, LD). Aus dem individuellen Quotienten LD : $ED_{-30\ mm\ Hg}$ lässt sich der therapeutische Index errechnen.

In einigen Fällen konnte die tödliche Dosis nicht ermittelt werden, da die Löslichkeitsgrenze niedriger als die Verträglichkeitsgrenze lag (dies ist durch das Zeichen > in der Tabelle kenntlich gemacht).

b) Prüfung auf β-blockierende Aktivität:

Kaninchen wurden in Holzkäfigen fixiert, die Herzfrequenz wurde bei den wachen Tieren über Stichelektroden abgeleitet und auf einem Frequenzzahlgerät (Messzeit 15 sec) abgelesen. Über eine Ohrvene wurde zunächst 1 µg/kg Isoprenalin intravenös injiziert, was eine Erhöhung der Herzfrequenz von ca. 200 Schlägen/min auf 330 Schläge/min bewirkt. Anschliessend wurden die Prüfsubstanzen in steigender Dosierung (siehe Methode a) intravenös zugeführt und die Herzfrequenz nach Isoprenalin erneut ausgezählt. Die Hemmung der Isoprenalin-Tachykardie ist als β-Blockade anzusehen. Es wurde die Dosis der Prüfsubstanzen bestimmt, die den Anstieg der Isoprenalin-Tachykardie auf die Hälfte begrenzt ($HD_{50\%}$).

Die Daten aus beiden Versuchen sind in der Tabelle wiedergegeben. Die Berechnung der äquieffektiven Dosen ($DE_{-30\ mm\ Hg}$, $HD_{50\%}$), LD) sowie des Quotienten wurden auf logarithmischer Basis aus 4 – 6 Einzelversuchen vorgenommen.

Es wurden folgende Verbindungen getestet:

Beispiel 1a) = 4-⟨2-Hydroxy-3-[4-()-2-benzimidazolinon-hydrochlorid

Beispiel 1b) = 4-⟨2-Hydroxy-3-[4-(]-propoxy⟩-2-benzimidazolinon-hydrochlorid

Beispiel 1f) = 4-⟨2-Hydroxy-3-[4-(2-pyridyloxymethyl)-piperidino]-propoxy⟩-2-benzimidazolinon-hydrochlorid

Beispiel 1g) = 4-⟨2-Hydroxy-3-[4-(]-propoxy⟩-2-benzimidazolinon-hydrochlorid

Beispiel 4a) = 4-[2-Hydroxy-3-(4-phenoxymethyl-piperidino)]-7-methyl-2-benz-imidazolinon-hydrochlorid

Beispiel 8b) = 4-⟨2-Hydroxy-3-[4-(2-methoxy-phenoxymethyl)-piperidino]-propoxy⟩-oxindol-acetat

Beispiel 8c) = 4-⟨2-Hydroxy-3-[4-(4-hydroxy-phenoxymethyl)-piperidino]-propoxy⟩-oxindol

Beispiel 10b) = 4-⟨2-Hydroxy-3-[4-(2-methoxy-phenoxymethyl)-piperidino]-propoxy⟩-carbazol-hydrochlorid

Beispiel 10c) = 4-⟨2-Hydroxy-3-[4-(2-methyl-phenoxymethyl)-piperidino]-propoxy⟩-carbazol-hydrochlorid

Beispiel 10d) = 4-⟨2-Hydroxy-3-[4-(3-methyl-phenoxymethyl)-piperidino]-propoxy⟩-carbazol-hydrochlorid

β-blockierende und vasodilatierende Aktivität der neuen Substanzen

| Substanz | $DE_{-30\ mm\ Hg}$ (vasodilatierende Akt.) (µg/kg i.v.) | $HD_{50\%}$ (β-blockierende Akt.) (µg/kg i.v.) | LD (tox. Dosis) (µg/kg i.v.) | LD / $ED_{-30\ mm\ Hg}$ (individuell log. errechnet) |
|---|---|---|---|---|
| Beispiel 1a) | 880 | 1156 | 16000 | 18 |
| Beispiel 1b) | 300 | 866 | 56570 | 19 |
| Beispiel 1f) | 1520 | 885 | >32000 | >21 |
| Beispiel 1g) | 920 | 171 | >11200 | >12 |
| Beispiel 4a) | 240 | 1018 | >26000 | >106 |
| Beispiel 8b) | 2370 | 360 | 32000 | 13 |
| Beispiel 8c) | 418 | 829 | 20159 | 48 |

| Substanz | $DE_{-30\ mm\ Hg}$ (vasodilatierende Akt.) (µg/kg i.v.) | $HD_{50\%}$ (β-blockierende Akt.) (µg/kg i.v.) | LD (tox. Dosis) (µg/kg i.v.) | LD / $ED_{-30\ mm\ Hg}$ (individuell log. errechnet) |
|---|---|---|---|---|
| Beispiel 10b) | 1600 | 217 | >10763 | >7 |
| Beispiel 10c) | 780 | 1031 | 16127 | 21 |
| Beispiel 10d) | 950 | 368 | 12800 | 14 |

PATENTANSRÜCHE

1. Piperidinopropylderivate der allgemeinen Formel I

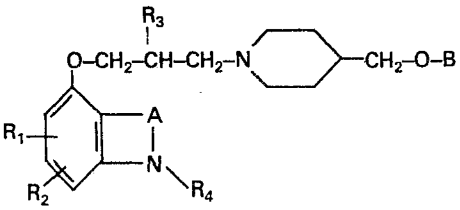

(I),

in der

$R_1$ und $R_2$, die gleich oder verschieden sein können, jeweils ein Wasserstoffatom oder eine $C_1$–$C_6$-Alkylgruppe oder gemeinsam einen $C_2$–$C_4$-Alkylenrest,

$R_3$ Wasserstoff oder eine Gruppe $-O-R_5$, wobei $R_5$ für Wasserstoff oder eine Acetyl-, Pivaloyl- und Benzoylrest steht,

$R_4$ Wasserstoff oder den Formylrest,

A die Gruppe $X_1=Y_1$, wobei $X_1$ und $Y_1$ gleich oder verschieden sein können und jeweils ein Stickstoffatom oder eine Gruppe $\underset{\displaystyle R_6}{-\overset{|}{C}=}$ bedeuten,

in der $R_6$ für Wasserstoff, eine $C_1$–$C_6$-Alkylgruppe, die gegebenenfalls durch eien Gruppe $-O-R_5$ substituiert ist, eine Carboxyl- oder $C_1$–$C_6$-Alkoxycarbonylgruppe steht, die Gruppe $X_2$–$Y_2$, in der $X_2$ eine $>CH_2$-Gruppe oder eine Gruppe $>N-R_7$, wobei $R_7$ für Wasserstoff oder eine $C_1$–$C_6$-Alkylgruppe steht, und $Y_2$ eine $>CH_2$-Gruppe oder eine Gruppe $>C=Z$, wobei Z für Sauerstoff- oder Schwefelatom steht, darstellt, oder die Gruppe $-CR_8=CR_9-$, wobei $R_8$ und $R_9$ zusammen eine $-CH=CH-CH=CH$-Brücke darstellen, mit der Massgabe, dass jeweils $Y_1$ bzw. $Y_2$ mit dem Rest $\rangle N-R_4$ der allgemeinen Formel I verbunden ist, und

B eine Pyridyl-, Pyrimidyl-, Benzimidazolinonyl-, Benzimidazolinyl-, Benztriazolyl-, Indazolyl-, Benzodioxolanyl- oder Carbazolylrest oder, falls A $X_2$–$Y_2$ oder $-CR_8=CR_9-$ darstellt, auch einen Phenylrest, die gegebenenfalls ein- oder mehrfach durch ein Halogen, eine Hydroxy-oder $C_1$–$C_6$-Alkylgruppe, die gegebenenfalls einen Hydroxy- oder Carboxamido-Substituenten trägt, eine $C_1$–$C_6$-Alkoxy-, Amino-, Carboxamido-, $C_1$–$C_6$-Alkylcarbonylamino-, oder $C_1$–$C_6$-Alkylsulfonylaminogruppen substituiert sind,

bedeuten, sowie deren pharmakologisch unbedenklichen Salze.

2. Verbindungen gemäss Anspruch 1 mit den dort angegebenen Bedeutungen der Substituenten $R_1$, $R_2$, $R_3$, $R_4$ und B, in denen der Benzolring mit der Gruppierung $\underset{\displaystyle R_4}{-A-\overset{}{N}-}$ einen Benzimidazolinon-(2)-, Benzimidazolinthion-(2)-, Oxindol-, Indazol-, Carbazol-, Benztriazol-, Benzimidazol-, Indolin- oder Indol-Ring bildet.

3. Verfahren zur Herstellung von Piperidinopropylderivaten der allgemeinen Formel I

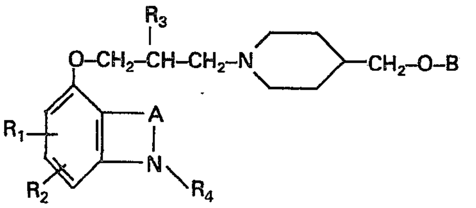

(I),

in der

$R_1$ und $R_2$, die gleich oder verschieden sein können, jeweils ein Wasserstoffatom oder eine $C_1$–$C_6$-Alkylgruppe oder gemeinsam einen $C_2$–$C_4$-Alkylenrest,

$R_3$ Wasserstoff oder eine Gruppe $-O-R_5$, wobei $R_5$ für Wasserstoff oder einen Acetyl-, Pivaloyl- und Benzoylrest steht,

$R_4$ Wasserstoff oder den Formylrest,

A die Gruppe $X_1=Y_1$, wobei $X_1$ und $Y_1$ gleich oder verschieden sein können und jeweils ein Stickstoffatom oder eine Gruppe $\underset{\displaystyle R_6}{-\overset{|}{C}=}$ bedeuten,

in der $R_6$ für Wasserstoff, eine $C_1$–$C_6$-Alkylgruppe, die gegebenenfalls durch eine Gruppe $-O-R_5$ substituiert ist, eine Carboxyl- oder $C_1$–$C_6$-Alkoxycarbonylgruppe steht, die Gruppe $X_2$–$Y_2$, in der $X_2$ eine $>CH_2$-Gruppe oder eine Gruppe $>N-R_7$, wobei $R_7$ für Wasserstoff oder eine $C_1$–$C_6$-Alkylgruppe steht, und $Y_2$ eine $>CH_2$-Gruppe oder eine Gruppe $>C=Z$, wobei Z für ein Sauerstoff- oder Schwefelatom steht, darstellt, oder die Gruppe $-CR_8=CR_9-$, wobei $R_8$ und $R_9$ zusammen eine $-CH=CH-CH=CH$-Brücke darstellen, mit der Massgabe, dass jeweils $Y_1$ bzw. $Y_2$ mit dem Rest $\rangle N-R_4$ der allgemeinen Formel I verbunden ist, und

B einen Pyridyl-, Pyrimidyl-, Benzimidazolinonyl-, Benzimidazolinyl-, Benztriazolyl-, Indazolyl-, Benzodioxolanyl- oder Carbazolylrest oder, falls A $X_2$–$Y_2$ oder $-CR_8=CR_9-$ darstellt, auch einen Phenylrest die gegebenenfalls ein- oder mehrfach durch ein Halogen, eine Hydroxy- oder $C_1$–$C_6$-Al-

kylgruppe, die gegebenenfalls einen Hydroxy- oder Carboxamido-Substituenten trägt, eine $C_1$–$C_6$-Alkoxy-, Amino-, Carboxamido-, $C_1$–$C_6$-Alkylcarbonylamino-, oder $C_1$–$C_6$-Alkylsulfonylaminogruppen substituiert sind, bedeuten, sowie deren pharmakologisch unbedenklichen Salze, dadurch gekennzeichnet, dass man in an sich bekannter Weise entweder

a) eine Verbindung der allgemeinen Formel II

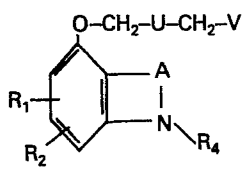

(II)

mit einer Verbindung der allgemeinen Formel III

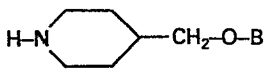

(III)

umsetzt, in denen $R_1$, $R_2$, $R_4$, A und B die oben angegebene Bedeutung haben, V einen reaktiven Rest darstellt und U für die Gruppe >C=O, >CH–$R_3$ oder >CH–O–E steht, wobei $R_3$ die oben angegebene Bedeutung hat und E zusammen mit V eine Einfachbindung bildet, und falls U die Gruppe >C=O bedeutet, anschliessend reduziert, oder

b) eine Verbindung der allgemeinen Formel IV

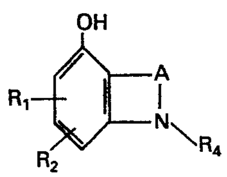

(IV)

mit einer Verbindung der allgemeinen Formel V

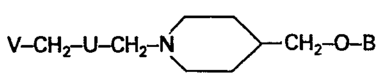

(V)

umsetzt, in denen $R_1$, $R_2$, $R_4$, A, B, U und V die oben angegebene Bedeutung haben, und falls U die Gruppe >C=O bedeutet, anschliessend reduziert,

oder

c) eine Verbindung der allgemeinen Formel VI

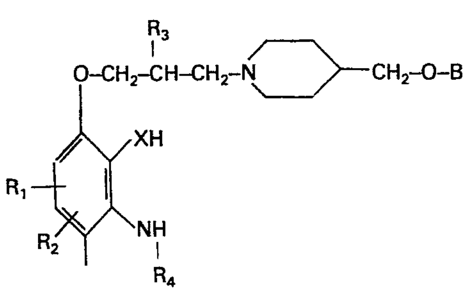

(VI),

in der $R_1$, $R_2$, $R_3$, $R_4$ und B die oben angegebene Bedeutung haben und X $HX_1$ oder $X_2$ vorstellt, für den Fall, dass A eine $-X_1=Y_1$-Gruppe bedeutet, eine Verbindung der Formel VI, in der X die Gruppe $HX_1$ darstellt, mit einer Verbindung der allgemeinen Formel VII a

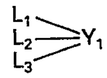

(VII a),

in der $Y_1$ die oben angegebene Bedeutung hat, $L_1$ ein Wasserstoffatom, die Hydroxygruppe oder einen reaktiven Rest T, $L_2$ ein Wasserstoffatom oder einen reaktiven Rest T und $L_3$ ein Wasserstoffatom oder zusammen mit $L_2$ ein Sauerstoffatom darstellt,

oder

für den Fall, dass A eine $-X_2-Y_2$-Gruppe bedeutet, eine Verbindung der Formel VI, in der X die Gruppe $X_2$ darstellt, mit einer Verbindung der allgemeinen Formel VII b

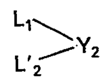

(VII b),

in der $L_1$ und $Y_2$ die oben angegebene Bedeutung haben und $L'_2$ ein Wasserstoffatom oder einen reaktiven Rest T bedeutet,

umsetzt und cyclisiert,

oder

d) eine Verbindung der allgemeinen Formel VIII

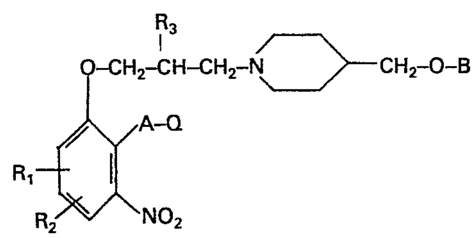

(VIII),

in der $R_1$, $R_2$, $R_3$, A und B die oben angegebene Bedeutung haben und Q eine abspaltbare Gruppe darstellt,

reduziert und cyclisiert,

und anschliessend Verbindungen der allgemeinen Formel I, in denen $R_3$ die Hydroxygruppe bedeutet, gegebenenfalls acyliert, einen Rest $R_6$ oder einen Substituenten im Rest B gewünschtenfalls in einen anderen Substituenten umwandelt und gegebenenfalls die erhaltenen Verbindungen der allgemeinen Formel I in ihre pharmakologisch verträglichen Salze überführt.

4. Arzneimittel, enthaltend eine Verbindung gemäss Ansprüchen 1–2 sowie übliche Träger- und Hilfsstoffe.

5. Verwendung von Verbindungen gemäss Ansprüchen 1–2 zur Herstellung von Arzneimitteln zur Bekämpfung von Herz- und Kreislauferkrankungen.

6. Verbindungen gemäss Ansprüchen 1–2 zur Verwendung bei der Bekämpfung von Herz- und Kreislauferkrankungen.

**Revendications**

1. Dérivés de pipéridinopropyle de formule générale I

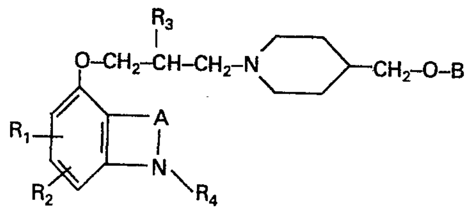

(I),

dans laquelle,

$R_1$ et $R_2$, pouvant être identiques ou différents sont chacun un atome d'hydrogène ou un groupe alkyle $C_1$–$C_6$ ou forment ensemble un reste alkylène $C_2$–$C_4$,

$R_3$ est de l'hydrogène ou un groupe $-O-R_5$, où $R_5$ est de l'hydrogène ou un reste acétyle, pivaloyle ou benzoyle,

$R_4$ est de l'hydrogène ou le reste formyle,

A est soit le groupe $X_1=Y_1$ où $X_1$ et $Y_1$ peuvent être identiques ou différents et sont chacun un atome d'azote ou un groupe $-\underset{\displaystyle R_6}{\overset{|}{C}}=$, dans lequel $R_6$ est de l'hydrogène, un groupe alkyle $C_1$–$C_6$ éventuellement substitué par un groupe $-O-R_5$, un groupe carboxyle ou alcoxy carbonyle $C_1$–$C_6$, soit le groupe $X_2$–$Y_2$ dans lequel $X_2$ est un groupe $>CH_2$ ou un groupe $>N-R_7$, où $R_7$ est de l'hydrogène ou un groupe alkyle $C_1$–$C_6$, et $Y_2$ groupe $>CH_2$ ou un groupe $>C=Z$ où Z est un atome d'oxygène ou un atome de soufre, soit le groupe $-CR_8=CR_9-$, où $R_8$ et $R_9$ forment ensemble un pont $-CH=CH-CH=CH-$, sous la condition que chaque fois $Y_1$ ou $Y_2$ est relié au reste $>N-R_4$ de la formule générale I, et

B est un reste pyridyle, pyrimidyle, benzimidazolinonyle, benzimidazolinyle, benzotriazolyle, indazolyle, benzodioxolanyle ou carbazolyle ou, dans le cas où A est $X_2$–$Y_2$ ou $-CR_8=CR_9-$, aussi un reste phényle substitué éventuellement une ou plusieurs fois par un halogène, un groupe hydroxyle ou alkyle $C_1$–$C_6$, portant éventuellement un substituant hydroxyle ou carboxamido, ou par un groupe alcoxy $C_1$–$C_6$, amino, carboxamido, alkylcarbonylamino $C_1$–$C_6$, ou alkylsulfonylamino $C_1$–$C_6$, ainsi que leurs sels pharmacologiquement tolérables.

2. Composés selon la revendication 1 dans lesquels les substituants $R_1$, $R_2$, $R_3$, $R_4$ et B ont les significations indiquées, dans lesquels le noyau benzène forme avec le groupe $-A-\underset{\displaystyle R}{\overset{}{N}}$ un noyau benzimidazolinon-(2), benzimidazolin-thion-(2), oxindole, indazole, carbazole, benzotriazole, benzimidazole, indoline ou indole.

3. Procédé pour la préparation de dérivés de pipéridinopropyle de formule générale I

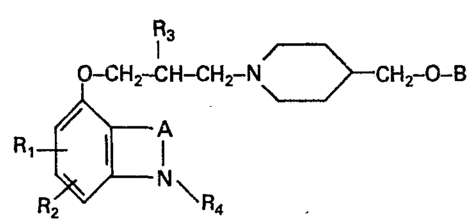

(I),

dans laquelle:

$R_1$ et $R_2$, pouvant être identiques ou différents sont chacun un atome d'hydrogène ou un groupe alkyle $C_1$–$C_6$ ou forment ensemble un reste alkylène $C_2$–$C_4$,

$R_3$ est de l'hydrogène ou un groupe $-O-R_5$, où $R_5$ est de l'hydrogène ou un reste acétyle, pivaloyle ou benzoyle,

$R_4$ est de l'hydrogène ou le reste formyle,

A est soit le groupe $X_1=Y_1$ où $X_1$ et $Y_1$ peuvent être identiques ou différents et sont chacun un atome d'azote ou un groupe $-\underset{\displaystyle R_6}{\overset{|}{C}}=$, dans lequel $R_6$ est de l'hydrogène, un groupe alkyle $C_1$–$C_6$ éventuellement substitué par un groupe $-O-R_5$, un groupe carboxyle ou alcoxy carbonyle $C_1$–$C_6$, soit le groupe $X_2$–$Y_2$ dans lequel $X_2$ est un groupe $>CH_2$ ou un groupe $>N-R_7$, où $R_7$ est de l'hydrogène ou un groupe alkyle $C_1$–$C_6$, et $Y_2$ un groupe $>CH_2$ ou un groupe $>C=Z$ où Z est un atome d'oxygène ou un atome de soufre, soit le groupe $-CR_8=CR_9-$, où $R_8$ et $R_9$ forment ensemble un pont $-CH=CH-CH=CH-$, sous la condition que chaque fois $Y_1$ ou $Y_2$ est relié au reste $>N-R_4$ de la formule générale I, et

B est un reste pyridyle, pyrimidyle, benzimidazolinonyle, benzimidazolinyle, benzotriazolyle, indazolyle, benzodioxolanyle ou carbazolyle ou, dans le cas où A est $X_2$–$Y_2$ ou $-CR_8=CR_9-$, aussi un reste phényle substitué éventuellement une ou plusieurs fois par un halogène, un groupe hydroxyle ou alkyle $C_1$–$C_6$, portant éventuellement un substituant hydroxyle ou carboxamido, ou par un groupe alcoxy $C_1$–$C_6$, amino, carboxamido, alkylcarbonylamino $C_1$–$C_6$, ou alkylsulfonylamino $C_1$–$C_6$, ainsi que leurs sels pharmacologiquement tolérables; caractérisé en ce que de façon en soi connue, soit

a) on fait réagir un composé de formule générale II

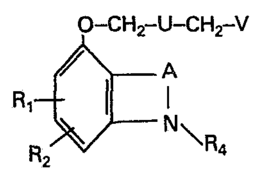

(II)

avec un composé de formule générale III

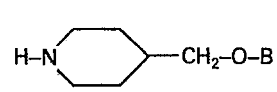

(III)

dans lesquelles:

$R_1$, $R_2$, $R_4$, A et B ont la signification ci-dessus indiquée, V est un reste réactif et U représente le groupe $>C=O$, $>CH-R_3$ ou $>CH-O-E$, où $R_3$ a la signification ci-dessus donnée et E forme ensemble avec V une liaison simple, et dans le cas où U est le groupe $>C=O$ on effectue ensuite une réduction, soit:

b) on fait réagir un composé de formule générale IV

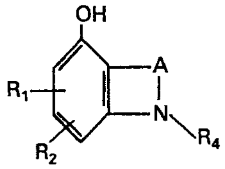

(IV)

avec un composé de formule générale V

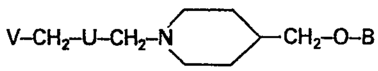

(V)

dans lesquelles $R_1$, $R_2$, $R_4$, A, B, U et V ont la signification ci-dessus indiquée et dans le cas où U est le groupe $>C=O$, on effectue ensuite une réduction, soit:

c) on transforme et on cyclise un composé de formule générale VI

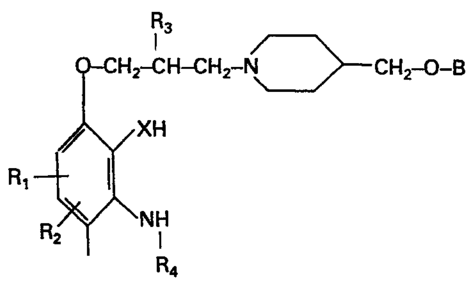

(VI),

dans laquelle $R_1$, $R_2$, $R_3$, $R_4$ et B ont la signification ci-dessus indiquée, et X représente $HX_1$ ou $X_2$, c'est-à-dire que dans le cas où A est un groupe $-X_1=Y_1-$, X est le groupe $HX_1$, avec un composé de formule générale VIIa

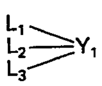

(VII a),

dans laquelle $Y_1$ a la signification ci-dessus indiquée,

$L_1$ est un atome d'hydrogène, le groupe hydroxyle ou un reste réactif T,

$L_2$ est un atome d'hydrogène ou un reste réactif T et $L_3$ est un atome d'hydrogène ou représente ensemble avec $L_2$ un atome d'oxygène, et dans le cas où A est un groupe $-X_2-Y_2$, X est le groupe $X_2$, avec un composé de formule générale VII b

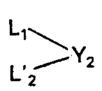

(VII b),

dans laquelle $L_1$ et $Y_1$ ont la signification donnée ci-dessus et $L'_2$ est un atome d'hydrogène ou un reste réactif T, soit:

d) on réduit et on cyclise un composé de formule générale VIII

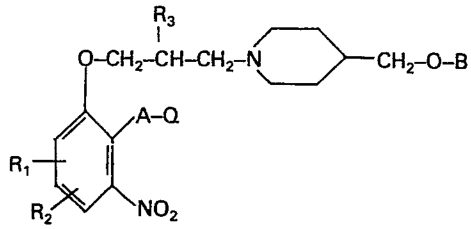

(VIII),

dans laquelle $R_1$, $R_2$, $R_3$, A et B ont la signification donnée ci-dessus et Q est un groupe éliminable, et en ce qu'éventuellement ensuite on effectue une acylation sur les composés de formule générale I dans lesquels $R_3$ est le groupe hydroxyle, on transforme si on le désire un reste $R_6$ ou un substituant dans le reste B en un autre substituant, ou on transforme éventuellement les composés obtenus de formule générale I en leurs sels pharmacologiquement tolérables.

4. Médicaments contenant un composé défini dans l'une quelconque des revendications 1 à 2 ainsi que les substances de support et auxiliaires habituelles.

5. Utilisation des composés selon les revendications 1 à 2 pour la fabrication de médicaments pour le traitement des maladies du cœur et de la circulation.

6. Composés selon les revendications 1 à 2 pour l'utilisation dans le traitement des maladies du cœur et de la circulation.

**Claims**

1. Piperidinopropyl derivatives of the general formula I

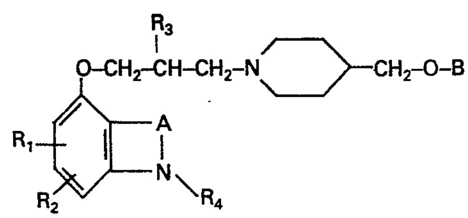

(I),

in which $R_1$ und $R_2$, which can be the same or different, each represent a hydrogen atom or a $C_1$–$C_6$ alkyl group or together a $C_2$–$C_4$ alkylene radical, $R_3$ a hydrogen atom or a group $-O-R_5$, whereby $R_5$ stands for hydrogen or an acetyl, pivaloyl or benzoyl radical, $R_4$ hydrogen or the formyl radical, A is the group $X_1=Y_1$, whereby $X_1$ and $Y_1$ can be the same or different and each represents a nitrogen atom or a group $-\underset{R_6}{\overset{|}{C}}=$, in which $R_6$ stands for hydrogen, a $C_1$–$C_6$ alkyl radical, which is optionally substituted by a group $-O-R_5$, a carboxyl or $C_1$–$C_6$ alkoxycarbonyl group; the group $-X_2-Y_2-$, in which $X_2$ represents a $=CH_2$ group or a $=N(R_7)$-group, whereby $R_7$ stands for hydrogen or a $C_1$–$C_6$ alkyl group, and $Y_2$ a $=CH_2$ group or a group $=C(=Z)$-, whereby Z stands for an oxygen or sulphur atom; or the group $-CR_8=CR_9-$, whereby $R_8$ and $R_9$ together represent a $-CH=CH-CH=CH$-bridge, with the proviso that, in each case. $Y_1$ or $Y_2$ is connected to the radical $>N-R_4$ of the general

formula I, and B represents a pyridyl, pyrimidyl, benzimidazolinonyl, benzimidazolinyl, benztriazolyl, indazolyl, benzdioxolanyl or carbazolyl radical or, if A represents $X_2$–$Y_2$ or $-CR_8{=}CR_9-$, also a phenyl radical, which are optionally substituted one or more times by a halogen, a hydroxyl or $C_1$–$C_6$ alkyl group, which optionally carries a hydroxyl or carboxamido substituent, a $C_1$–$C_6$ alkoxy, amino, carboxamido, $C_1$–$C_6$ alkylcarbonylamino or $C_1$–$C_6$ alkylsulphonylamino group; as well as their pharmacologically acceptable salts.

2. Compounds according to claim 1, with the theregiven meanings of the substituents $R_1$, $R_2$, $R_3$, $R_4$ and B, in which the benzene ring forms, with the grouping $-A-\underset{\displaystyle R_4}{\underset{|}{N}}-$, a benzimidazolin-2-one, benzimidazolin-2-thione, exindole, indazole, carbazole, benztriazole, benzimidazole, indoline or indole ring.

3. Process for the preparation of piperidinopropyl derivatives of the general formula I

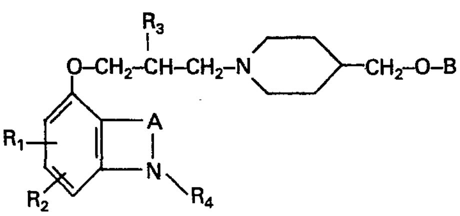
(I),

in which $R_1$ and $R_2$, which can be the same or different, each represent a hydrogen atom or a $C_1$–$C_6$ alkyl group or together a $C_2$–$C_4$ alkylene radical, $R_3$ hydrogen or a group $-O-R_5$, whereby $R_5$ stands for hydrogen or an acetyl, pivaloyl or benzoyl radical, $R_4$ a hydrogen atom or the formyl radical, A the grou $X_1{=}Y_1$, whereby $X_1$ and $Y_1$ can be the same or different and each represents a nitrogen atom or a $-\underset{\displaystyle R_6}{\underset{|}{C}}=$ group, in which $R_6$ stands for hydrogen, a $C_1$–$C_6$ alkyl group, which is optionally substituted by a group $-O-R_5$, a carboxyl or $C_1$–$C_6$ alkoxycarbonyl group; the group $X_2$–$Y_2$ in which $X_2$ represents a $=CH_2$ group or a group $=N(R_7)$, whereby $R_7$ stands for hydrogen or a $C_1$–$C_6$ alkyl group, and $Y_2$ a $=CH_2$ group or a group $=C(=Z)$, whereby Z stands for an oxygen or sulphur atom; or the group $-CR_8{=}CR_9$-, whereby $R_8$ and $R_9$ together represent a $-CH{=}CH-CH{=}CH-$ bridge, with the proviso that, in each case, $Y_1$ or $Y_2$ is connected to the radical $>N-R_4$ of the general formula I, and B a pyridyl, pyrimidyl, benzimidazolinonyl, benzimidazolinyl, benztriazolyl, indazolyl, benzodioxolanyl or carbazolyl radical or, if A represents $X_2$–$Y_2$ or $-CR_8{=}CR_9-$, also a phenyl radical, which are optionally substituted one or more times by a halogen, a hydroxyl or $C_1$–$C_6$ alkyl group, which optionally carries a hydroxyl or carboxamido substituent, a $C_1$–$C_6$ alkoxy, amino, carboxamido, $C_1$–$C_6$ alkylcarbonylamino or $C_1$–$C_6$ alkylsulphonylamino group, as well as of their pharmacologically acceptable salts thereof, characterised in that, in per se known manner, one either

a) reacts a compound of the general formula II

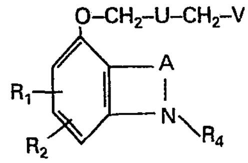
(II)

with a compound of the general formula III

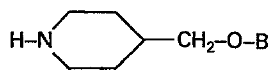
(III)

in which $R_1$, $R_2$, $R_4$, A and B have the above-given meaning, V represents a reactive residue and U stands for the group $>C{=}O$, $>CH-R_3$ or $>CH-O-E$, whereby $R_3$ has the above-given meaning and E, together with V, forms a single bond, and, if U signifies the group $>C{=}O$, subsequently reduces, or

b) reacts a compound of the general formula IV

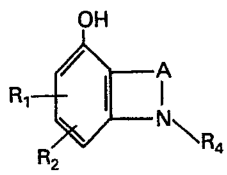
(IV)

with a compound of the general formula V

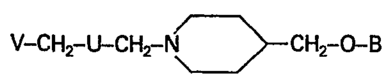
(V)

in which $R_1$, $R_2$, $R_4$, A, B, U and V have the above-given meaning, and, if U signifies the group $>C{=}O$, subsequently reduces; or

c) reacts and cyclises a compound of the general formula VI

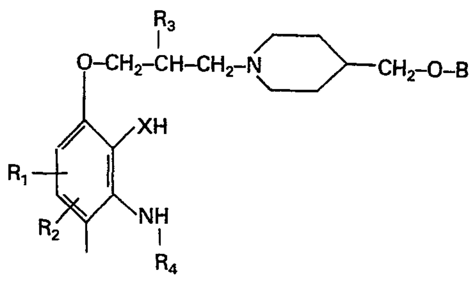
(VI),

in which $R_1$, $R_2$, $R_3$, $R_4$ and B have the above-given meaning and X represents $HX_1$ or $X_2$, for the case in which A signifies an $-X_1{=}Y_1$-group, a compound of the formula VI, in which X represents the group $HX_1$, with a compound of the general formula VIIa

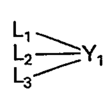
(VII a),

in which $Y_1$ has the above-given meaning, $L_1$ represents a hydrogen atom, the hydroxyl group or

a reactive residue T, $L_2$ a hydrogen atom or a reactive residue T and $L_3$ a hydrogen atom or, together with $L_2$, an oxygen atom; or, for the case in which A signifies an $-X_2-Y_2$-group, a compound of formula VI, in which X represents the group $X_2$, with a compound of the general formula VIIb

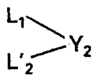

(VII b),

in which $L_1$ and $Y_2$ have the above-given meaning and $L_2'$ signifies a hydrogen atom or a reactive residue T, or

d) reduces and cyclises a compound of the general formula VIII

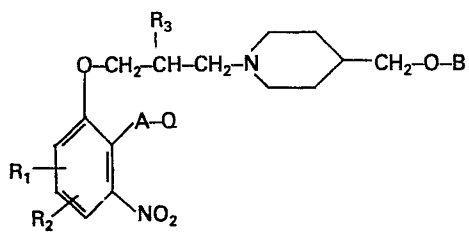

(VIII),

in which $R_1$, $R_2$, $R_3$, A and B have the above-given meaning and Q is a group which can be split off, and subsequently optionally acylates a compound of the general formula I, in which $R_3$ signifies the hydroxyl group, optionally converts a radical $R_6$ or a substituent in the radical B into another substituent and optionally converts the compounds obtained of the general formula I into their pharmacologically acceptable salts.

4. Medicaments containing a compound according to claim 1 or 2, as well as conventional carrier and adjuvant materials.

5. Use of compounds according to claim 1 or 2, for the preparation of medicaments for the combating of heart and circulatory diseases.

6. Compounds according to claim 1 or 2 for use in the combating of heart and circulatory diseases.